# EUROPEAN PATENT APPLICATION

(11) **EP 3 406 253 A1**
(43) Date of publication of application: **28.11.2018**
(21) Application number: 18172979.9
(22) Date of filing: 17.05.2018
(51) Int. Cl.: A61K 31/7088, C12N 15/113, A61K 39/395, A61P 35/00

(54) **INHIBITORS AND ANTAGONISTS OF HUMAN PYCR1**

(30) Priority: 24.05.2017 EP 17172756; 05.04.2018 EP 18165811
(71) Applicant: Bayer Aktiengesellschaft, 51373 Leverkusen (DE); Bayer Pharma Aktiengesellschaft, 13353 Berlin (DE); Agency for Science, Technology and Research, Singapore 138632 (SG)
(72) Inventor: GRÜNEWALD, Sylvia, 10557 Berlin (DE); STECKEL, Michael, 13088 Berlin (DE); HUSEMANN, Manfred, 16540 Hohen Neuendorf (DE); MEYER, Hanna, 13187 Berlin (DE); HAN, Weiping, 138667 Singapore (SG); DING, Zhaobing, 138667 Singapore (SG)
(74) Representative: BIP Patents

(57) **Abstract**

The present invention is related to an antagonist of PYCR1 for the treatment and/or prevention of a neoplastic disease.

## Description

The present invention provides inhibitors and antagonists of human PYCR1, for the treatment and/or prevention of a neoplastic disease. The invention provides inhibitors and antagonists in the form of antibodies, nucleic acids, aptamers, or small molecules. The invention also provides assays and screening technologies to find such inhibitors and antagonists.

### Background of the invention

Despite tremendous progress in the last 3 decades, cancer therapy is still a field with many unmet needs. There are cancer types which do not respond on established therapies, or respond poorly, with high recurrence rates or continued formation of metastases. This applies, *inter alia,* to tumors and cancers in the liver, the skin or the oesophagus.

With an estimated 700,000 new cases per year, hepatocellular carcinoma (HCC) represents the fifth commonest cancer and is the third leading cause of cancer death worldwide. HCC cases currently occur predominantly in East and Southeast Asia, and Sub-Saharan Africa due to Hepatitis C viral infection and are increasing in the Western nations, largely due to an increasing incidence of metabolic complications, such as non-alcoholic steatohepatitis related to obesity and type II diabetes. The 5-year survival rate remains below 12% due to limited treatment options (El-Serag 2011). Besides Sorafenib, an oral multikinase inhibitor approved for the treatment of HCC (Llovet, Ricci et al. 2008), there is no other pharmacological agent approved for therapy of HCC. However, due to the severe side effects and only a 2-3 month gain of life, the identification of additional effective therapeutic targets remains a top priority.

To meet increased energetic and anabolic needs of sustained cell growth, cancer cells undergo dramatic metabolic reprogramming (Boroughs and DeBerardinis 2015). The expression changes observed in cancer cells can be linked to the activity of multiple classic oncogenes and tumor suppressors. Overall, accumulating evidence demonstrates that changes in metabolism can confer a potent influence on tumor development and growth, enough so that it is now considered an emerging hallmark of cancer (Ward and Thompson 2012). These metabolic changes also hold the potential for therapeutic intervention. A notable advancement has been the targeting of changes in serine and glycine metabolism for certain cancers (Locasale, Grassian et al. 2011; Possemato, Marks et al. 2011).

Therefore, metabolic enzyme expression profiles in different HCC animal models were compared to identify metabolic pathways that could be targeted offering treatment options for HCC patients. *In vitro* gene silencing experiments revealed similar results for HCC, melanoma and oesophageal cancer cell lines.

### Summary of the invention

These and further objects are met with methods and means according to the independent claims of the present invention. The dependent claims are related to specific embodiments.

### Embodiments of the invention

Before the invention is described in detail, it is to be understood that this invention is not limited to the particular component parts or structural features of the devices or compositions described or process steps of the methods described as such devices and methods may vary. It is also to be understood that the terminology used herein is for purposes of describing particular embodiments only, and is not intended to be limiting. The mere fact that certain measures are recited in mutually different dependent claims does not indicate that a combination of these measures cannot be used to advantage. Any reference signs in the claims should not be construed as limiting the scope. It must be noted that, as used in the specification and the appended claims, the singular forms "a," "an" and "the" include singular and/or plural referents unless the context clearly dictates otherwise. Further, in the claims, the word "comprising" does not exclude other elements or steps. The mere fact that certain measures are recited in mutually different dependent claims does not indicate that a combination of these measures cannot be used to advantage.

It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the length of this specification manageable. It is further to be understood that the content of the prior art documents referred to herein is incorporated by reference, e.g., for enablement purposes, namely when e.g. a method is discussed details of which are described in said prior art document. This approach serves to keep the length of this specification manageable.

According to one aspect of the invention, an antagonist of PYCR1 for the treatment and/or prevention of a neoplastic disease is provided.

The method disclosed herein to determine such antagonist is so comprehensive and sufficiently disclosed that it allows the identification of such antagonist without the need to embark on an unduly burdensome research program, in particular when the skilled person recourses to common general knowledge. This is because the specification provides sufficient information on how to select such potentially active molecule both for its theoretical use to treat neoplastic disease, as well as for its therapeutic suitability.

Pyrroline-5-carboxylate reductase 1 (PYCR1) is a mitochondria-associated enzyme which belongs to the family of oxidoreductases. Preferably, said PYCR1 is human PYCR1. In humans, the enzyme is encoded by the *PYCR1* gene (PYCR1, UniProt: P32322). The enzyme catalyzes the NAD(P)H-dependent conversion of pyrroline-5-carboxylate to proline. The reverse reaction is catalyzed by proline dehydrogenase (PRODH).

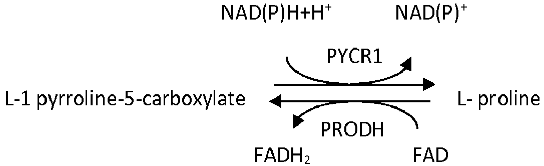

The enzyme forms a homopolymer and localizes to the mitochondrion. Alternate splicing of the gene results in different transcript variants encoding different isoforms, as disclosed in Entrez Gene: PYCR1 pyrroline-5-carboxylate reductase 1. Homozygous deletion or missense mutations of PYCR1 have been shown to cause skin disorders in humans like Autosomal recessive cutis laxa (ARCL) (Reversade et al (2009), Kretz et al (2011). It has further been shown that expression of PYCR1 is upregulated in a variety of tumor types (Roth et al).

However, upregulation does not mean that PYCR1 is causative for the genesis of cancer in general, or specific tumors in particular. Upregulation can also be the downstream consequence of upregulation of another gene, or caused by a general impairment of gene regulation as it frequently occurs in malignant tissues.

The inventors have for the first time shown that inhibition of PYCR1 can reduce proliferation in specific tumor models *in vitro* and *in vivo,* particularly in liver tumor, skin tumor, and esophageal tumor.

While these findings have been made with gene knockdown experiments, part of which were induced knockdowns in tumor xenograft mice, an extrapolation of these findings on potential effects of an antagonist or inhibitors of PYCR1, e.g., a suitable antibody, a suitable nucleic acid molecule, a suitable aptamer or a suitable small molecule, is plausible. The findings are quite surprising insofar as homozygous deletion or missense mutations of PYCR1 have been shown to cause skin disorders in humans like Autosomal recessive cutis laxa (ARCL). Hence, inhibition of PYCR1 has so far been associated with pathologic effects rather than with a therapeutic approach for treating cancer.

Accordingly, in one embodiment of the invention, a liver tumor, or a secondary tumor derived from a liver tumor. This includes hepatocarcinoma, hepatocellular carcinoma, cholangiocarcinoma, angiosarcoma and/or hepatoblastoma and other liver tumors.

In one other embodiment of the invention, the neoplastic disease treated by the PYCR1 antagonist is a skin tumor, or a secondary tumor derived from a skin tumor. This includes melanoma, basal-cell skin cancer, squamous-cell skin cancer or merkel cell carcinoma, and other skin tumors.

In one other embodiment of the invention, the neoplastic disease treated by the PYCR1 antagonist is an oesophageal tumor, or a secondary tumor derived from an oesophageal tumor.

As used herein the term "antagonist of PYCR1 "refers to regulators or effectors or modulators of PYCR1 nucleic acids that inhibit, decrease or prevent the intracellular response of PYCR1 polypeptides or PYCR1 nucleic acids (including mRNA and genomic DNA). The term is used interchangeably with the term "inhibitor".

According to one embodiment, the antagonist inhibits, directly or indirectly, PYCR1-mediated formation of L-Proline. In this context, the term "directly" means that the inhibitor according to the invention directly affects the enzymatic activity of PYCR1, e.g., by binding to the latter and/or steric hindrance. The term "indirectly" means that the inhibitor affects the gene expression of PYCR1, e.g., by sequence-specific interaction with the mRNA or the encoding genomic DNA.

According to another embodiment of the invention, the antagonist is a monoclonal antibody, or a target-binding fragment or derivative thereof retaining target binding capacities that specifically binds to one or more isoforms of the PYCR1 protein.

As used herein, the term "monoclonal antibody (mAb)", shall refer to an antibody composition having a homogenous antibody population, i.e., a homogeneous population consisting of a whole immunoglobulin, or a fragment or derivative thereof retaining target binding capacities. Particularly preferred, such antibody is selected from the group consisting of IgG, IgD, IgE, IgA and/or IgM, or a fragment or derivative thereof retaining target binding capacities.

As used herein, the term "fragment" shall refer to fragments of such antibody retaining target binding capacities, e.g.
- a CDR (complementarity determining region)
- a hypervariable region,
- a variable domain (Fv)
- an IgG heavy chain (consisting of VH, CH1, hinge, CH2 and CH3 regions)
- an IgG light chain (consisting of VL and CL regions), and/or
- a Fab and/or F(ab)₂.

As used herein, the term "derivative" shall refer to protein constructs being structurally different from, but still having some structural relationship to, the common antibody concept, e.g., scFv, Fab and/or F(ab)₂, as well as bi-, tri- or higher specific antibody constructs, and further retaining target binding capacities. All these items are explained below.

Other antibody derivatives known to the skilled person are Diabodies, Camelid Antibodies, Nanobodies, Domain Antibodies, bivalent homodimers with two chains consisting of scFvs, IgAs (two IgG structures joined by a J chain and a secretory component), shark antibodies, antibodies consisting of new world primate framework plus non-new world primate CDR, dimerised constructs comprising CH3+VL+VH, and antibody conjugates (e.g. antibody or fragments or derivatives linked to a toxin, a cytokine, a radioisotope or a label). These types are well described in literature and can be used by the skilled person on the basis of the present disclosure, without adding further inventive activity.

As discussed above, PYCR1 is sufficiently specified to enable a skilled person to make a monoclonal antibody thereagainst. Routine methods encompass hybridoma, chimerization/ humanization, phage display/transgenic mammals, and other antibody engineering technologies.

Methods for the production of a hybridoma cell are disclosed in Köhler & Milstein (1975). Essentially, e.g., a mouse is immunized with a human PYCR1 protein, following B-cell isolation and fusion with a myeloma cell.

Methods for the production and/or selection of chimeric or humanised mAbs are known in the art. Essentially, e.g., the protein sequences from the murine anti PYCR1 antibody which are not involved in target binding are replaced by corresponding human sequences. For example, US6331415 by Genentech describes the production of chimeric antibodies, while US6548640 by Medical Research Council describes CDR grafting techniques and US5859205 by Celltech describes the production of humanised antibodies.

Methods for the production and/or selection of fully human mAbs are known in the art. These can involve the use of a transgenic animal which is immunized with human PYCR1, or the use of a suitable display technique, like yeast display, phage display, B-cell display or ribosome display, where antibodies from a library are screened against human PYCR1 in a stationary phase.

In vitro antibody libraries are, among others, disclosed in US6300064 by MorphoSys and US6248516 by MRC/Scripps/Stratagene. Phage Display techniques are for example disclosed in US5223409 by Dyax. Transgenic mammal platforms are for example described in EP1480515A2 by TaconicArtemis.

IgG, scFv, Fab and/or F(ab)₂ are antibody formats well known to the skilled person. Related enabling techniques are available from the respective textbooks.

As used herein, the term "Fab" relates to an IgG fragment comprising the antigen binding region, said fragment being composed of one constant and one variable domain from each heavy and light chain of the antibody

As used herein, the term "F(ab)₂" relates to an IgG fragment consisting of two Fab fragments connected to one another by disulfide bonds.

As used herein, the term "scFv" relates to a single-chain variable fragment being a fusion of the variable regions of the heavy and light chains of immunoglobulins, linked together with a short linker, usually serine (S) or glycine (G). This chimeric molecule retains the specificity of the original immunoglobulin, despite removal of the constant regions and the introduction of a linker peptide.

Modified antibody formats are for example bi- or trispecific antibody constructs, antibody-based fusion proteins, immunoconjugates and the like. These types are well described in literature and can be used by the skilled person on the basis of the present disclosure, with adding further inventive activity.

Finding a suitable antibody, or fragment or derivative, that is capable of acting as an antagonist of PYCR1, e.g., by binding to its active centre, is hence a matter of routine for the skilled person, based on the public availability of the amino acid sequences of the different PYCR1 isoforms.

Polyclonal antibodies against PYCR1 for scientific research are commercially available, e.g., from Proteintech (20962-1-AP and 13108-1-AP), Abcam (ab72953), ThermoFischer (PA5-30141, PA5-26890), Sigma Aldrich (HPA047660, sab2104620), Novus Biologicals (H00005831-B01P and NBP1-57934)), hence demonstrating that the skilled person is capable of making a therapeutic antibody against PYCR1.

Because PYCR1 is an intracellular enzyme, the antibody or its fragment or derivative needs to be funneled or trafficked into the intracellular space. Routine technologies are available for these purposes, which are disclosed, *inter alia,* in Chen & Erlanger (2002), Berguig et al (2015), Meunier, Executive Interview, Thura et al (2016).

According to another embodiment of the invention, the antagonist comprises a first nucleic acid molecule that specifically binds to a second nucleic acid molecule, which second nucleic acid molecule encodes an isoform of the PYCR1 protein.

Said second nucleic acid molecule can be an mRNA transcribed from the gene encoding for the PYCR1 protein. Said second nucleic acid is devoid of introns, but due to alternative splicing different mRNAs transcribed from the gene encoding for the PYCR1 protein can differ from one another. In such case, the first nucleic acid molecule can be a siRNA (small interfering RNA) or a shRNA (short hairpin RNA).

siRNAs are short artificial RNA molecules which can be chemically modified to enhance stability. Because siRNAs are double-stranded, the principle of the 'sense' and the 'antisense' strand also applies. The sense strands have a base sequence identical to that of the transcribed mRNA and the antisense strand has the complementary sequence. Technically, a siRNA molecule administered to a patient is bound by an intracellular enzyme called Argonaut to form a so-called RNA-induced silencing complex (RISC). The antisense strand of the siRNA guides RISC to the target mRNA, where the antisense strand hybridizes with the target mRNA, which is then cleaved by RISC. In such way, translation of the respective mRNA is interrupted. The RISC can then cleave further mRNAs. Delivery technologies are e.g. disclosed in Xu and Wang (2015). Finding a suitable sequence for the siRNA is a matter of routine for the skilled person, based on the public availability of the different mRNA isoforms of PYCR1.

shRNA is an artificial RNA molecule with a tight hairpin turn that can be used to silence target gene expression via RNA interference (RNAi). shRNA can be delivered to cells, e.g., by means of a plasmid or through viral or bacterial vectors. shRNA is an advantageous mediator of RNAi in that it has a relatively low rate of degradation and turnover. The respective plasmids comprise a suitable promoter to express the shRNA, like a polymerase III promoter such as U6 and H1 or a polymerase II promoter. Once the plasmid or vector has integrated into the host genome, the shRNA is transcribed in the nucleus. The product mimics pri-microRNA (pri-miRNA) and is processed by Drosha. The resulting pre-shRNA is exported from the nucleus by Exportin 5. This product is then processed by Dicer and loaded into the RNA-induced silencing complex (RISC), after which the same silencing follows as in siRNA. Finding a suitable sequence for the shRNA is a matter of routine for the skilled person, based on the public availability of the different mRNA isoforms of PYCR1.

Said second nucleic acid molecule can also be a genomic DNA comprised in the gene encoding for the PYCR1 protein. Said gene comprises several non-coding introns, hence its sequence differs from the sequence of the mRNA or the cDNA disclosed herein.

In such case, the first nucleic acid molecule can be the guide RNA of a CRISPR Cas system (see, e.g., Jinek et al (2012)), which guide RNA comprises a target-specific crRNA ("small interfering CRISPR RNA") capable of hybridizing with a genomic strand of the PYCR1 gene (or, the first nucleic acid molecule can be the crRNA alone). The guide RNA/crRNA is capable of directing the Cas enzyme, which is an endonuclease, to the PYCR1 gene, where the Cas enzyme carries out sequence specific strand breaks. By creating one or more double strand breaks, the PYCR1 gene hence can be silenced. To use said system for *in vivo gene silencing of PYCR1,* e.g., in different cells of a tumor, a dedicated delivery technology is required, which comprise a delivery vehicle such as lipid nanoparticles, as for example discussed in Yin et al (2016). Finding a suitable sequence for the crRNA comprised in the guide RNA is a matter of routine for the skilled person, based on the public availability of the genomic sequence of the PYCR1 gene.

In another embodiment, said first nucleic acid molecule can also the guide RNA of a CRISPR Cpf system (Zetsche et al (2015)), which guide RNA comprises a target-specific crRNA ("small interfering CRISPR RNA"). Similar to CRISPR Cas, the guide RNA is capable of directing the Cpf enzyme, which is an endonuclease, to the PYCR1 gene. As regards technical considerations, e.g., delivery for *in vivo* applications and finding of the suitable sequence for the first nucleic acid molecule, similar aspects as with CRISPR Cas apply.

Further embodiments of the CRISPR technology are currently under development, with different endonucleases. However, all these approaches use a target-specific RNA (the guide RNA or crRNA as in CRISPR Cas) that hybridizes with a target sequence. In all these cases, the target-specific RNA qualifies as the first nucleic acid molecule in the meaning of the preferred embodiment discussed herein . As regards technical considerations, e.g., delivery for *in vivo* applications and finding of the suitable sequence for the first nucleic acid molecule, similar aspects as with CRISPR Cas apply.

According to another embodiment of the invention, the antagonist is an aptamer that specifically binds to one or more isoforms of the PYCR1 protein, and/or inhibits the activity thereof.

Aptamers are oligonucleotides that have specific binding properties for a pre-determined target. They are obtained from a randomly synthesized library containing up to 10¹⁵ different sequences through a combinatorial process named SELEX ("Systematic Evolution of Ligands by EXponential enrichment"). Aptamer properties are dictated by their 3D shape, resulting from intramolecular folding, driven by their primary sequence. An aptamer3D structure is exquisitely adapted to the recognition of its cognate target through hydrogen bonding, electrostatic and stacking interactions. Aptamers generally display high affinity (K_{d} about micromolar for small molecules and picomolar for proteins).

An overview on the technical repertoire to generate target specific aptamers is given, e.g., in Blind and Blank (2015). Aptamers can also be delivered into the intracellular space, as disclosed in Thiel & Giangrande (2010).

Finding a suitable aptamer that is capable of acting as an antagonist of PYCR1, e.g., by binding to its active centre, is hence a matter of routine for the skilled person, based on the public availability of the amino acid sequences of the different PYCR1 isoforms.

According to another embodiment of the invention, the antagonist is a polypeptide or protein fragment, such as a synthesized polypeptide and a stapled peptide, that specifically binds to one or more isoforms of the PYCR1 protein, and/or inhibits the activity thereof.

An overview on the technical repertoire to generate stapled peptides is given, e.g., in Blind and Blank (2015) and Walensky and Bird (2014).

Finding a suitable polypeptide or protein fragment that is capable of acting as an antagonist of PYCR1, e.g., by binding to its active centre, is hence a matter of routine for the skilled person, based on the public availability of the amino acid sequences of the different PYCR1 isoforms.

According to another embodiment of the invention, the antagonist is a small molecule that specifically binds to one or more isoforms of the PYCR1 protein, and/or inhibits the activity thereof.

Said antagonist can for example be found by means of a PYCR1 inhibition assay, where the impact of a candidate molecule on the PYCR1-catalyzed transformation from L-Proline to L-1 pyrroline-5-carboxylate or L-1 pyrroline-5-carboxylate to L-Proline is determined. An example for such assay may look as follows:

### PYCR1 Inhibition Assay

The assay is based on the ability of PYCR1 to catalyze the oxidation of L-proline to L-1 pyrroline-5-carboxylate which occurs concomitant with the reduction of the cosubstrate NAD⁺ to its reduced form NADH+H⁺. The amount of NADH+H⁺ was quantified using the NAD(P)H-GloTM kit from Promega (catalogue number G9062). Components of the kit generate a luciferase-based luminescence signal the intensity of which is linearly proportional to the amount of NADH+H⁺ generated by the PYCR1 reaction and consequently the PYCR1 activity. The luminescence intensity was taken as the measure for the amount of NADH+H⁺ generated by PYCR1. Inhibition of PYCR1 results in a decreased amount of NADH+H⁺ as compared to an uninhibited control reaction and a consequently decreased luminescence intensity. Luminescence intensity was measured using a ViewLux Imager (PerkinElmer). The data were normalised (enzyme reaction without inhibitor = 0 % inhibition, all other assay components but no enzyme = 100 % inhibition) and IC₅₀ values were calculated using Genedata Screener® software.
1. 40 nl Test compound (dissolved and serially diluted in DMSO at final concentrations during the enzymatic reaction ranging from 6.9E-09 M to 4.5E-05 M) is added to the well of a white 1536-well microtiter plate using an acoustic dispenser (Echo Liquid Handler from Labcyte Inc.)
2. Add 2.0 µl PYCR1 full length recombinant enzyme (aa 1-319) with a 6His-tag at the N-terminus expressed in *E. coli* and purified by affinity chromatography on Nickel-sepharose (IMAC) and by gel filtration (SEC) in assay buffer (final concentration during the enzymatic reaction is 4.2 nM)
3. Preincubation of enzyme and test compound for approximately 15 min at room temperature
4. Add 2.0 µl Substrate mix in assay buffer (5 mM L-proline + 0.5 mM NAD⁺ final concentration during the enzymatic reaction)
5. 45 min PYCR1 reaction at room temperature (linear range of the enzymatic reaction)
6. Add 1.0 µl Stopping agent in assay buffer (2 mM ZnCl₂ final concentration)
7. Incubate for 15 min at room temperature
8. Add 2.0 µl NAD(P)H-Glo™ reagent (Promega)
9. Incubate for 30 - 150 min at room temperature for generation of the persistent luminescence signal
10. Measure luminescence intensity in a ViewLux Imager (PerkinElmer)

### Assay buffer:

100 mM Tris, pH 8.5
30 mM MgCl₂
0.001% Pluronic F-127
0.1% Bovine gamma-globuline (BGG)
1 mM T-CEP

Based on the above description, in particular when using the said assays, the skilled person is able to identify suitable PYCR1 antagonists or inhibitors. Examples of small molecular PYCR1 antagonists or inhibitors identified with said PYCR1 Inhibition Assay are shown in the following table, with the IC₅₀ indicated as determined in the PYCR1 Inhibition Assay.

| **Compound** | **Name** | **IC₅₀** | **CAS number** |
|---|---|---|---|
| 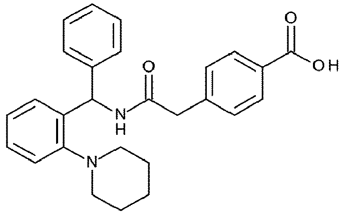 | 4-(2-oxo-2-{[phenyl(2-piperidin-1-ylphenyl)methyl]amino}ethyl)benzoic acid | 7.3 µM | 83901-40-0 |
| 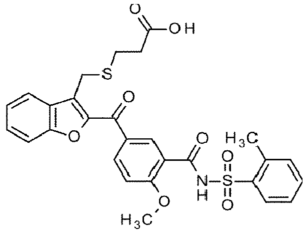 | 3-[({2-[(4-methoxy-3-{[(2-methylphenyl)sulfonyl]carbamoyl}phenyl)ca rbonyl]-1-benzofuran-3-yl}methyl)sulfanyl]propanoic acid | 2.9 µM | 151024-32-7 |
| 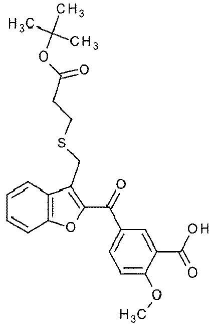 | 5-[(3-{[(3-tert-butoxy-3-oxopropyl)thio]methyl}-1-benzofuran-2-yl)carbonyl]-2-methoxybenzoic acid | 8.5 µM | 151024-29-2 |
| 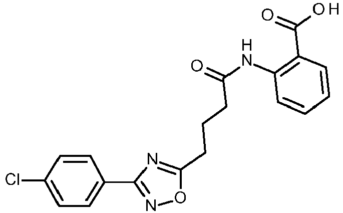 | 2-({4-[3-(4-chlorophenyl)-1,2,4-oxadiazol-5-yl]butanoyl}amino)benzoic acid | 8.0 µM | 845889-90-9 |

Generally, an antagonist or inhibitor identified by an assay described herein can be further validated for therapeutic effect by administration to a tumor or tumor model in which the *PYCR1* gene is overexpressed and/or there is excess of PYCR1 protein. A decrease in tumor growth confirms therapeutic utility of the modulator. Prior to treating a patient with the antagonist or inhibitor, the likelihood that the patient will respond to treatment can be diagnosed by obtaining a tumor sample from the patient, and assaying for expression of *PYCR1.* The expression data for the gene can also be used as a diagnostic marker for disease progression. The assay can be performed by expression analysis, by an antibody directed to the gene target or its expression product, or by any other available detection method.

According to another embodiment of the invention, the PYCR1 protein to which the antibody, aptamer or small molecule binds comprises a consensus sequence according to SEQ ID No 10. Said consensus sequence refers to SEQ ID Nos 6 - 8, which are the different isoforms of the PYCR1 enzymes.

According to another embodiment of the invention, the PYCR1 protein to which the antibody, aptamer or small molecule binds comprises a sequence according to any of SEQ ID Nos 6 - 8.

According to another embodiment of the invention, the nucleic acid encoding an isoform of the PYCR1 protein comprises a consensus sequence according to SEQ ID No 9. Said consensus sequence refers to SEQ ID Nos 1 - 5, which are the different transcription variants of the PYCR1 encoding gene.

According to another embodiment of the invention, the nucleic acid encoding an isoform of the PYCR1 protein comprises a sequence according to any of SEQ ID Nos 1 - 5.

According to another embodiment of the invention, the neoplastic disease is characterized by overexpression of *PYCR1* and/or reduced expression of *PRODH.* The term PRODH refers to proline dehydrogenase, which catalyzes the reverse reaction of PYCR1. Preferably, said PRODH is human PRODH (UniProtKB - O43272).

As used herein, the terms "overexpression of a gene" and "reduced expression of a gene" refer to expression patterns and/or expression levels of a gene normally not occurring under natural conditions, e.g. in a healthy tissue. The respective comparative expression patterns or levels can e.g. be taken from predetermined average expression patterns or levels from tissue databases or the like.

According to another embodiment of the invention, the neoplastic disease is characterized by excessive levels of PYCR1 protein (in the tumor/tumor cells).

As used herein, the terms "excess of a protein" and "excessive level of a protein" or the like refer to levels or amounts of a protein normally not occurring under natural conditions, e.g. in a healthy tissue. The respective comparative levels or amounts can e.g. be taken from predetermined average levels, levels/amounts from tissue databases, levels/amounts from non-tumor tissue (e.g. healthy tissue) or the like.

According to another aspect of the invention, the use of the antagonist (for the manufacture of a medicament) in the treatment of a human or animal subject being diagnosed for, suffering from or being at risk of developing a neoplastic disease, or in the prevention of such condition, is provided.

The invention further provides a pharmaceutical composition comprising an antagonist according to the above description, a combination of such pharmaceutical composition and one or more further therapeutically active compounds, and a method for treating or preventing a disorder or condition associated with the undesired expression of PYCR1, comprising administering to a subject in need thereof an effective amount of the said antagonist, pharmaceutical composition or combination.

### Sequence Listing

A sequence listing is enclosed which discloses the following sequences:

| | |
|---|---|
| SEQ ID No 1 | NM_001282279.1: Homo sapiens pyrroline-5-carboxylate reductase 1 (PYCR1), transcript variant 3, mRNA. |
| SEQ ID No 2 | NM_001282280.1 Homo sapiens pyrroline-5-carboxylate reductase 1 (PYCR1),transcript variant 4, mRNA. |
| SEQ ID No 3 | NM_001282281.1 Homo sapiens pyrroline-5-carboxylate reductase 1 (PYCR1),transcript variant 5, mRNA. |
| SEQ ID No 4 | NM_006907.3 Homo sapiens pyrroline-5-carboxylate reductase 1 (PYCR1),transcript variant 1, mRNA. |
| SEQ ID No 5 | NM_153824.2 Homo sapiens pyrroline-5-carboxylate reductase 1 (PYCR1), transcript variant 2, mRNA. |
| SEQ ID NO 6 | NP_001269209 pyrroline-5-carboxylate reductase 1, mitochondrial isoform 1 [Homo sapiens]. |
| SEQ ID NO 7 | NP_001269210.1 pyrroline-5-carboxylate reductase 1, mitochondrial isoform 5 [Homo sapiens]. |
| SEQ ID NO 8 | NP_722546.1 pyrroline-5-carboxylate reductase 1, mitochondrial isoform 2 [Homo sapiens]. |
| SEQ ID NO 9 | consensus sequence of SEQ ID Nos 1 - 5 |
| SEQ ID NO 10 | consensus sequence of SEQ ID Nos 6 - 8 |
| SEQ ID NO 11 | Rat PYCR1.1 shRNA: GCTGCCATAAAGAAGACTGTA |
| SEQ ID NO 12 | Rat PYCR1.2 shRNA: GAACCTGACACCCCACAACAA |
| SEQ ID NO 13 | Rat PYCR1.3 shRNA: GGCAGCCAAGATGCTACTAGA |

### Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Any reference signs should not be construed as limiting the scope. All amino acid sequences disclosed herein are shown from N-terminus to C-terminus; all nucleic acid sequences disclosed herein are shown 5'->3'.

### Examples

The experiments shown herein clearly support PYCR1 as a target whose inhibition provides a therapy option for HCC. *PYCR1* is overexpressed in rat and mouse HCC models compared to normal and regenerating liver as well as in human HCC cell lines compared to human hepatocytes, suggesting a role in tumorigenesis. Suppression of *PYCR1* in tumor cell lines that show increased PYCR1 was found to cause a dramatic decrease in tumor cell proliferation. Short hairpin RNAs (shRNAs) that inhibit *PYCR1* expression caused a change in morphology of MH3924A cells and extensively reduced tumor growth of subcutaneous xenografts generated with shRNA transfected MH3924A cells. Moreover, doxycycline-inducible *PYCR1* shRNAs caused reduction of tumor growth in orthotopic MH3924A xenograft models in either the preventive setting where PYCR1 knockdown is induced one day after cell inoculation or in the therapeutic setting where PYCR1 knockdown is induced after tumor formation, i.e. 7 days after cell inoculation to degrees consistent with their capacity to suppress PYCR1.

Based on the knockdown experiments shown herein, the suitability of a
- monoclonal antibody, or a target-binding fragment or derivative thereof retaining target binding capacities, that specifically binds to an isoform of the PYCR1 protein,
- a first nucleic acid molecule that specifically binds to a second nucleic acid molecule, which second nucleic acid molecule encodes an isoform of the PYCR1 protein, and/or
- an aptamer that specifically binds to an isoform of the PYCR1 protein
- a small molecule that specifically binds to an isoform of the PYCR1 protein
to achieve similar physiologic effects become immediately plausible, hence suggesting a suitability thereof to serve as an antagonist of PYCR1 for the treatment and/or prevention of a neoplastic disease.

All animal experimental protocols were approved by the Institutional Animal Care and Use Committee of the Biomedical Research Council of Singapore, carried out according to the guidelines for the care and use laboratory animals and BRC IACUC guidelines for cancer research in mice and rats.

### Example 1: PCR array to characterize gene expression in amino acid metabolic pathway

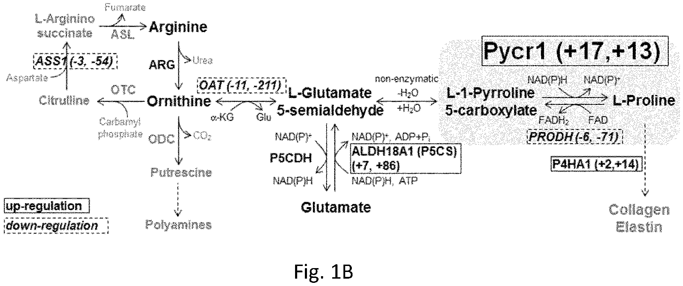

Mouse and rat amino acid metabolism I/II PCR arrays of 96-well plate format (cat. No.PAMM-130Z, cat. No.PARN-130Z) were purchased from Qiagen. Total RNAs from diethylnitrosamine-induced mouse liver tumors, orthotopic rat morris hepatoma tumors, regenerating liver tissue, and non-tumor liver tissues were extracted using RNeasy mini kit (Qiagen). 1.2 µg of total RNA was used for first strand synthesis with RT2 First Strand Kit (Qiagen), then first strand reaction mixed with RT2 SYBR green Mastermix was evenly distributed into 96 wells plate format containing the predispensed gene specific primer sets. Quantitative real-time PCR was performed according to RT2 Profiler PCR Array Handbook in an ABI StepOnePlus instrument and data were normalized to the 5 housekeeping genes (ACTB, B2M, GAPDH, HSP90ab1, GUSB) using the ΔΔCT method . The PCR conditions were 95°C for 10 min for initial enzyme activation and 40 cycles of amplification at 95°C for 15s and 60°C for 1min. Ct values were exported for web-based data analysis at http://pcrdataanalysis.sabiosciences.com/pcr/arrayanalysis.php. In DEN-induced mouse tumors and orthotopic rat MH3924A tumors the genes for the proline biosynthesis enzymes ALDH18A1 and PYCR1 were upregulated and those for the proline degradation enzymes PRODH and OAT were downregulated compared to normal liver tissue and regeneration liver tissue (Fig. 1B).

### Example 2: PYCR1 expression analysis by Array Northern

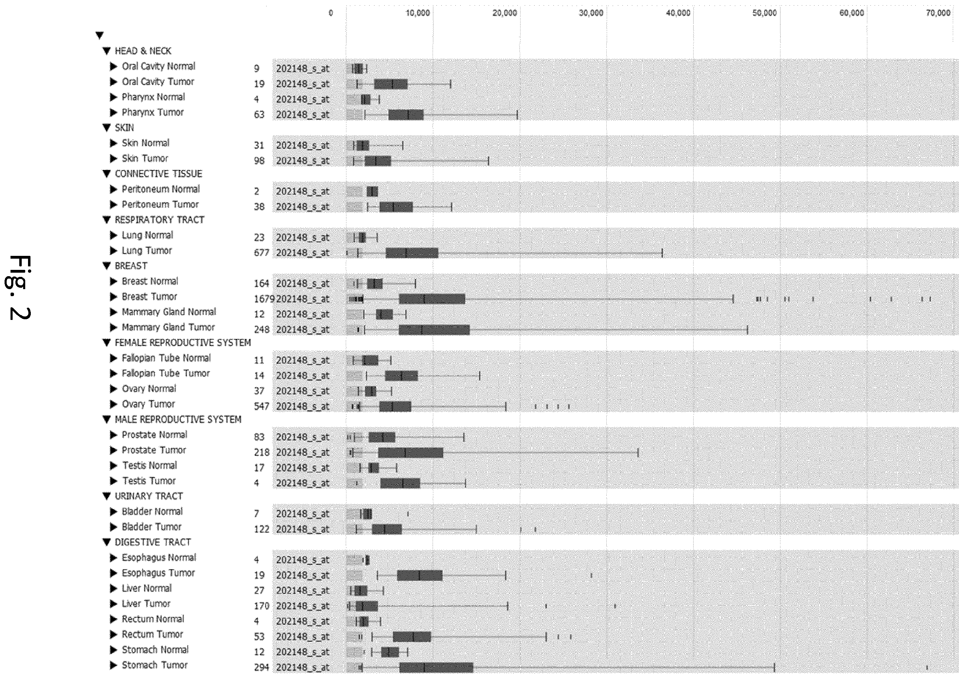

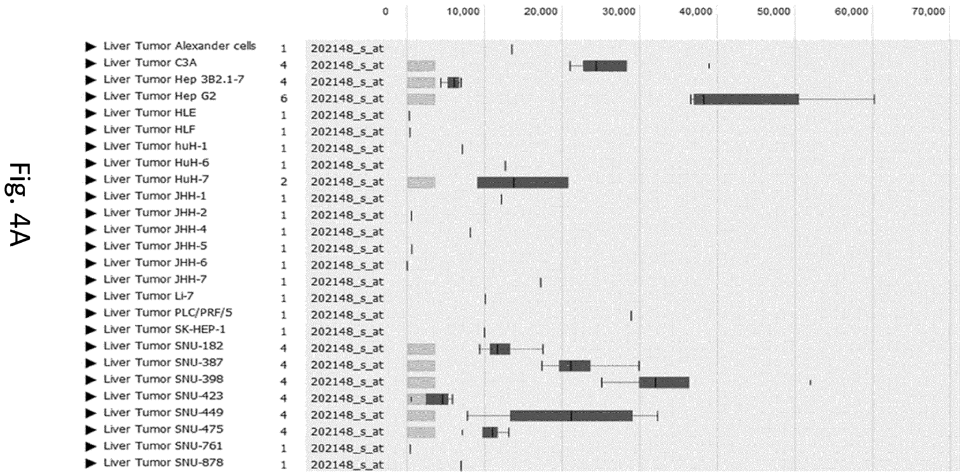

Array Northern is a proprietary application providing an overview of the expression of genes across healthy and diseased tissues. Array Northern is based on a compilation of Affymetrix gene expression experiments (HG-U133_Plus_2 array) from in-house studies and publicly available studies which were downloaded from Gene Expression Omnibus (GEO, https://www.ncbi.nlm.nih.gov/geo/) and ArrayExpress (https://www.ebi.ac.uk/arrayexpress/). Affymetrix CEL files were processed with the Affymetrix MAS5 algorithm and median-normalized. Samples were annotated with a controlled vocabulary for tissues, cell types and diseases. Array Northern generates box plots where each box summarizes the expression data of all samples of a predefined combination of sample annotation categories. The expression of the *PYCR1* gene probe 202148_s_at is displayed for the data collection "All Diseased, Tumor, Normal samples for AN 2012-09" and for the data collection "Human Cell Lines for AN 2012-08-14". Data for tumor indications with increased *PYCR1* expression are shown in Fig. 2 and *PYCR1* expression data in a human HCC cell line panel are shown in Fig. 4A. Expression data are transformed to a logarithmic scale. The length of whiskers is calculated using the logarithmized values. Whisker positions are transformed back to the linear scale using the exponential function. The center line of the box represents the median of all values. The lower and upper limit of the box represent the 25% and 75% quantiles, i.e. 50% of all values are within the box. To determine the position of whiskers, 1.5 times the box length is added at each side of the box as initial position of whiskers, and then whiskers are dragged towards the box until they hit a real value. All values outside the whiskers are outliers and are represented by small stand-alone lines.

### Example 3: Lentivirus-mediated shRNA knockdown studies with rat MH3924A cells

Short hairpin oligonucleotides targeting rat *Pycr1* were subcloned into lentiviral vector pLKO or pLKO-Tet via AgeI and EcoRI sites to generate respective lentiviral knockdown constructs. All the DNA constructs were validated by sequencing. The following oligonucleotides were used for vector construction: Rat PYCR1.1: GCT GCC ATA AAG AAG ACT GTA; Rat PYCR1.2: GAA CCT GAC ACC CCA CAA CAA; Rat PYCR1.3: GGC AGC CAA GAT GCT ACT AGA; Lentiviral particles were produced by co-transfection of HEK293T cells with lentiviral constructs and third generation packaging plasmids using the calcium phosphate transfection method. The supernatant containing the viral particles was collected 72 h after transfection, centrifuged at 1,000 g for 5 min and filtered with 0.45 µm filter. Collected lentiviruses were stored at -80°C.

Morris hepatoma cells (MH3924A) were cultured in Dulbecco's modified eagle medium (DMEM) media supplemented with 10% fetal bovine serum (FBS), 100 IU/ml penicillin, 100 µg/ml streptomycin. To assess cell proliferation, cells were seeded in T25 flasks and incubated in DMEM complete medium for 24 hours to allow attachment. Subsequently, seeding cells were infected with shRNA lentivirus in the presence of 8 µg/ ml of polybrene for about 7 hours, cells were grown for varying lengths of time, at the end of which they were detached by trypsinization and counted with Countess (Life Technologies, Carlsbad, USA).

### Example 4: Quantitative Real-Time PCR for gene expression analysis

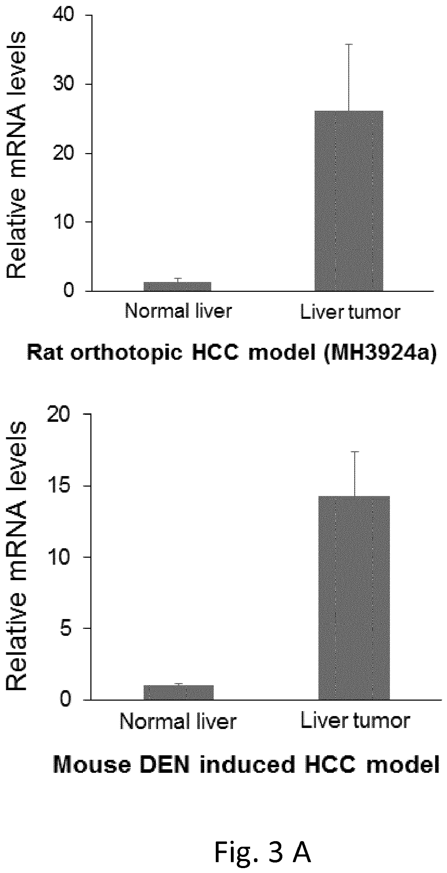

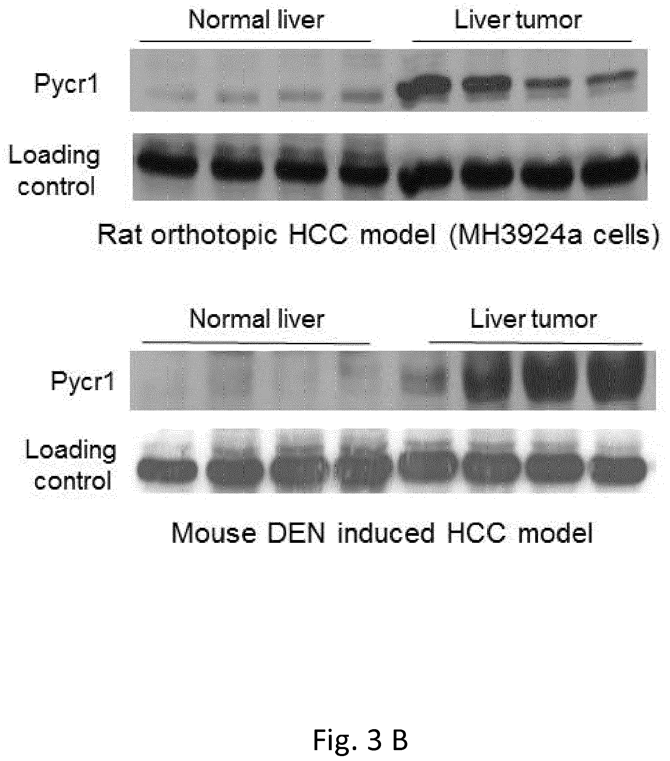

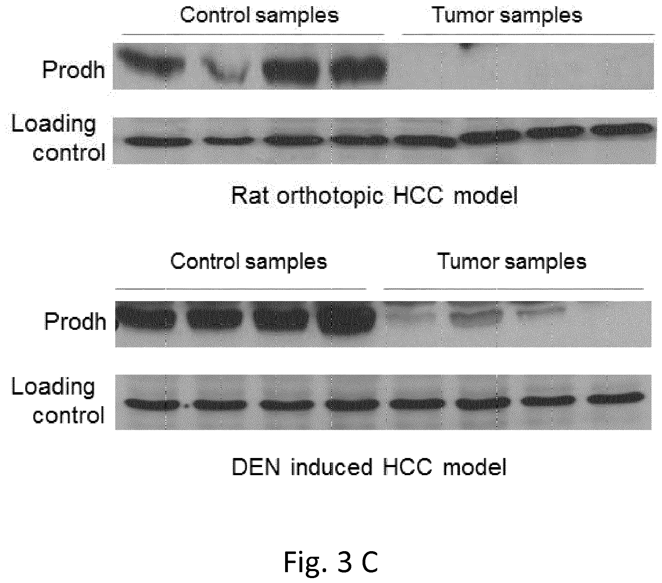

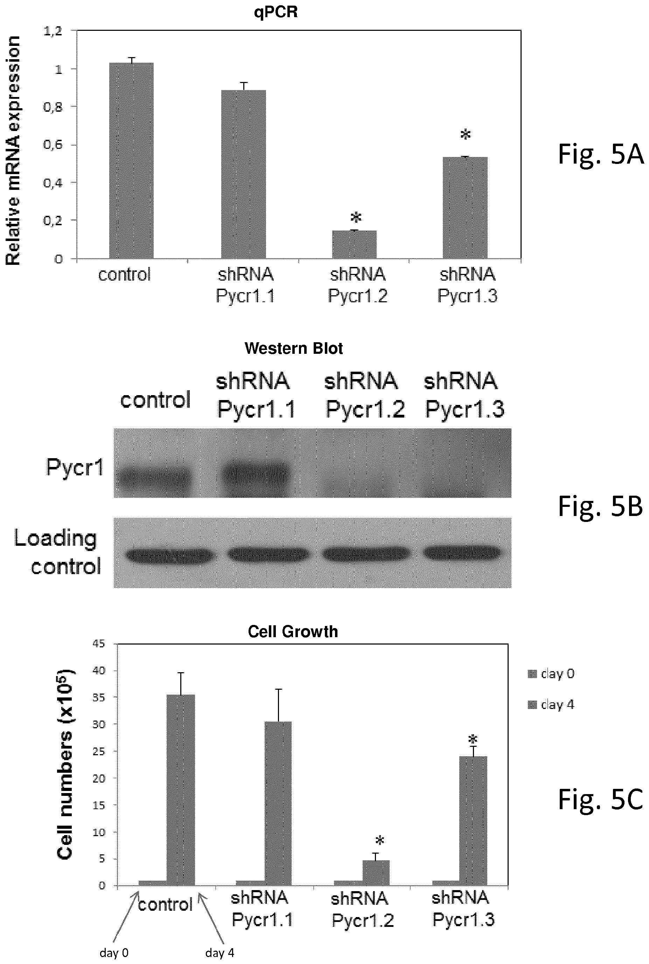

Total RNA was extracted from tumor and nontumor liver tissues according to the manufacturer's protocol (RNeasy kit, Qiagen, Germany). Total RNA (1.2 µg) was then reverse transcribed to cDNA by Superscript III reverse transcriptase (Invitrogen) with random hexamer in a total reaction volume of 20 µl. Real-time PCR reactions (in triplicates) were performed with the Fast Real-Time PCR System (Applied Biosystems, USA), using 1 µl of cDNA product in a reaction volume of 10 µl. The Taqman primers and probes (Applied Biosystemis) for target genes, *Pycr1* (Rn01751579_m1, Mm01205845 _m1), *Prodh* (Rn01404902_m1, Mm00448401_ml), the endogenous control genes were rat *Gapdh* (4352338E, VIC lavelled), mouse *Hprt*: (Mm 00446968 VIC labelled). The PCR conditions were 95°C for 20 s and 40 cycles of amplification at 95°C for 3 s and 60°C for 30 s. The transcript levels were expressed as fold of changes compared with non-tumor samples unless stated otherwise (Fig. 3 and 5).

### Example 5: Analysis of protein levels of PYCR1 in tumor tissue or cells

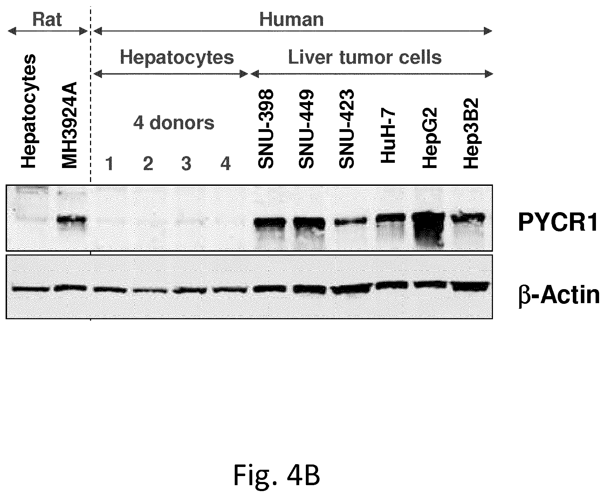

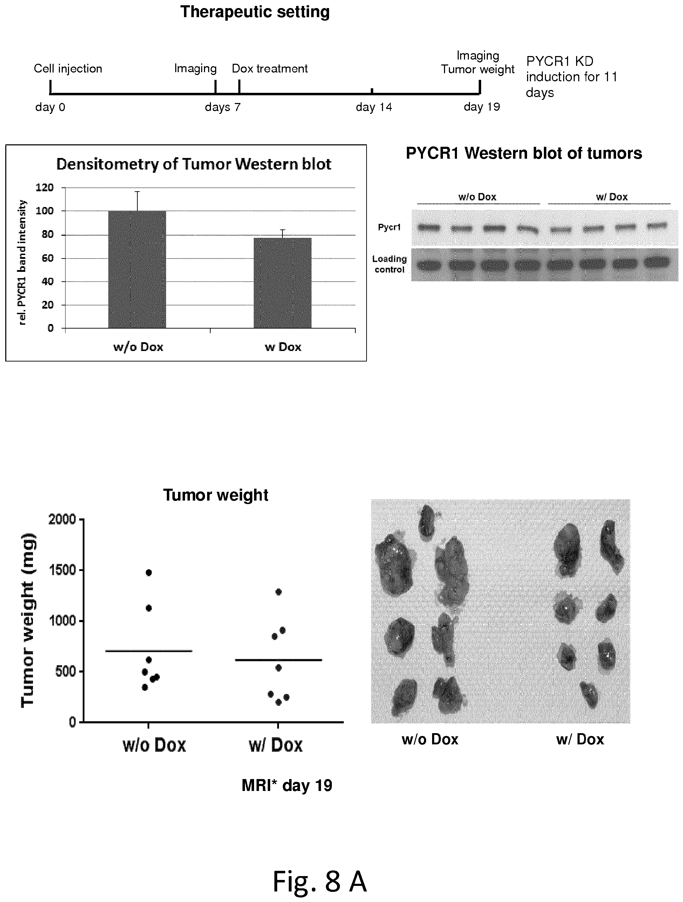

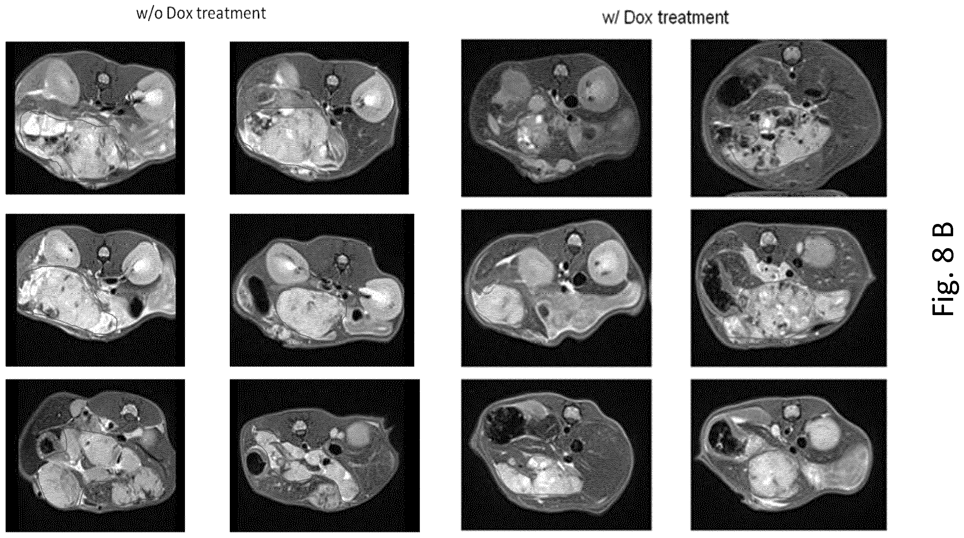

For tumor and non-tumor tissue proteins (Fig. 3 and 8), 50-75 µg of total protein extracts were further boiled at 950C for 5 min with 0.1%SDS, resolved in a 12% SDS-PAGE gel and transferred onto a nitrocellulose membrane. Primary antibodies PYCR1 (proteintech, 13108-1-AP), P5CS (proteintech, 17719-1-AP), PRODH (abcam, ab 93210) and GAPDH (Santa Cruz Biotechnology) were used at 1:1000 dilution. Target bands were detected by horseradish peroxidase-conjugated secondary antibody (GE healthcare). Protein bands were recorded on Xray film by reacting with ECL chemiluminescence reagents (GE healthcare). Similar procedures were used for cell line protein extracts (Fig. 4, 5 and 7).

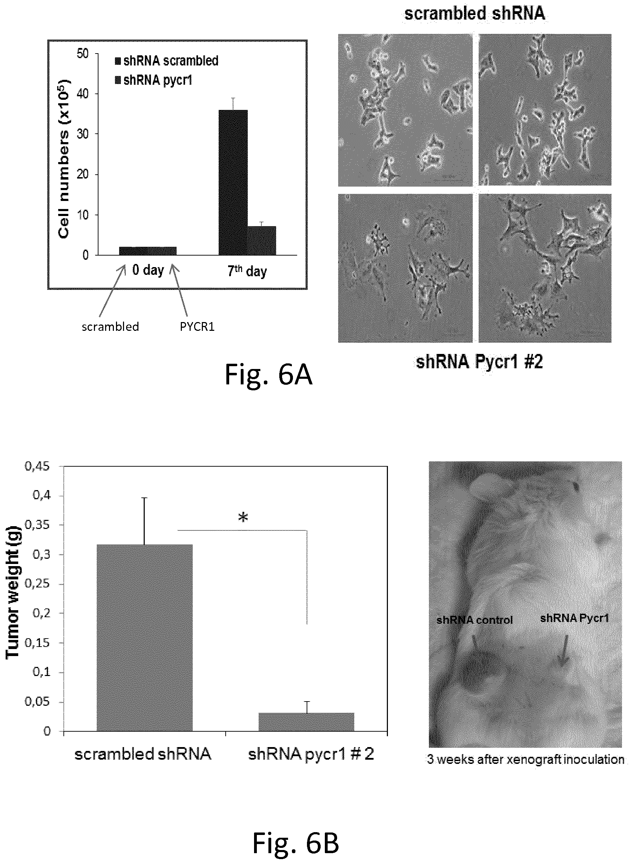

### Example 6: Subcutaneous MH3924A xenograft model (Fig. 6)

Female *scid* mice (InVivos, Singapore) were subcutaneously inoculated with 1 x 10⁶ rat MH3924A cells that were stably transfected with either shRNA Pycr1.2 or scrambled control shRNA. Mice were sacrificed and tumors excised 3 weeks later. Tumor weight was significantly reduced in the group that was inoculated with MH3924A cells stably expressing shRNA Pycr1.2 compared to control (n= 5).

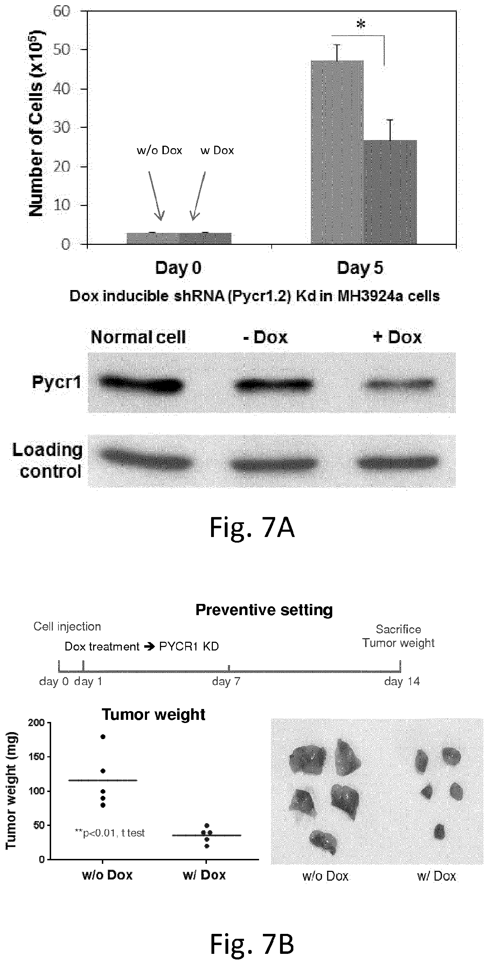

### Example 7: Orthotopic MH3924A xenograft models (Fig. 7-9)

The HCC model was created by the direct intrahepatic injection of rat MH3924A cells harboring inducible shRNA Pycr1.2 or scrambled shRNA constructs into *scid* mice (InVivos, Singapore). The mice were anesthetized and a midline incision was made to expose the liver. The cells were then slowly injected under the hepatic capsule into the upper left lobe of the liver using a 27-G needle. A pale, whitish color could be observed at the point of injection under the hepatic capsule. Gentle compression was applied with a cotton applicator to stop bleeding and reflux of the cells. The abdomen was closed with a 4-0 silk suture.

In the preventive setting (Fig. 7), shRNA expression was induced the day after cell injection by adding 1µg/ml doxycycline to the drinking water. Two weeks later, animals were sacrificed and tumors excised for analysis (n=5).

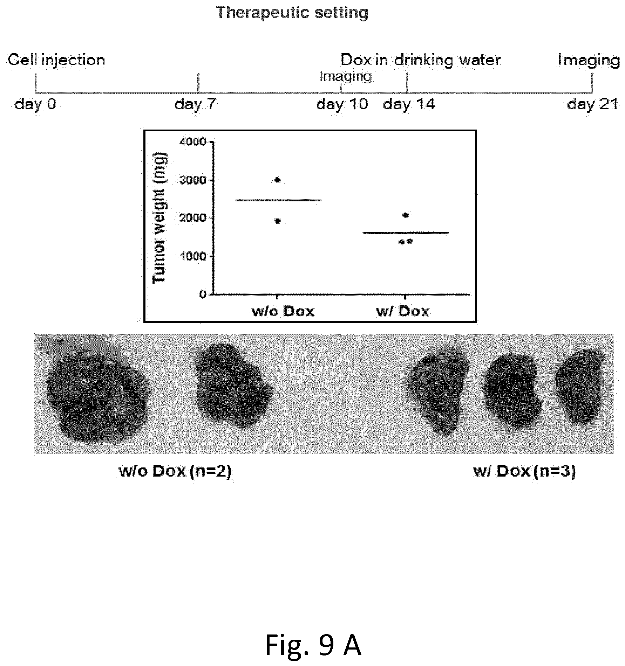

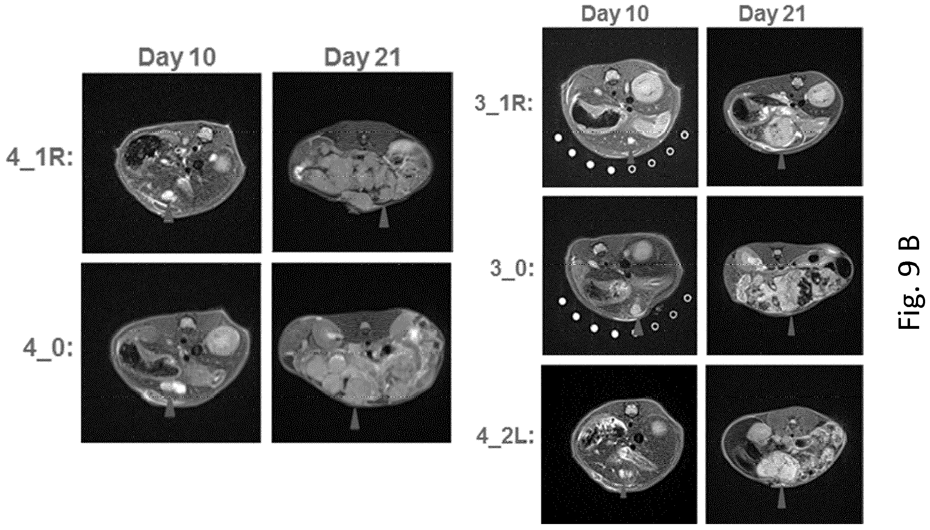

In the therapeutic setting, mice were subjected to liver MRI (magnetic resonance imaging) 7 days (1^{st} experiment, Fig. 8) or 10 days (2^{nd} experiment, Fig 9) after cell injection and shRNA expression was induced one or four days later by adding 1µg/ml doxycycline to the drinking water. On day 19 (1^{st} experiment) or 21 (2^{nd} experiment), animals were subjected to liver MRI and sacrificed. Tumors were lysed and analyzed for PYCR1 by Western blot.

### Example 8: DEN mouse HCC model

2-week-old male mice were injected intraperitoneally with 50 mg/kg body weight diethylnitrosamine (DEN). Mice were sacrificed at end of 8 months to collect liver tumor and perform gene expression analysis (Fig. 1B and 3).

### Example 9: Generation of liver regeneration model by 2/3 partial hepatectomy

Hepatectomy surgery was based on the method published by (Mitchell and Willenbring 2008). Wild type C57BL/6 mice or ACI rats were deeply anesthetized with Ketamine/xylazine via i.p. injection, laparotomy was performed to exposed liver, 4-0 silk threads were placed on the base of left lobe and median lobe, sutures were tied, then curved scissors were used to cut the tied left and median lobe. Liver regeneration was monitored on different time points, on 16th day, the liver weight almost recovered to its original size. To identify controlled cell proliferation, we collected liver tissues 48hr after hepatectomy to analyse gene expression, this was to avoid the acute response soon after hepatectomy.

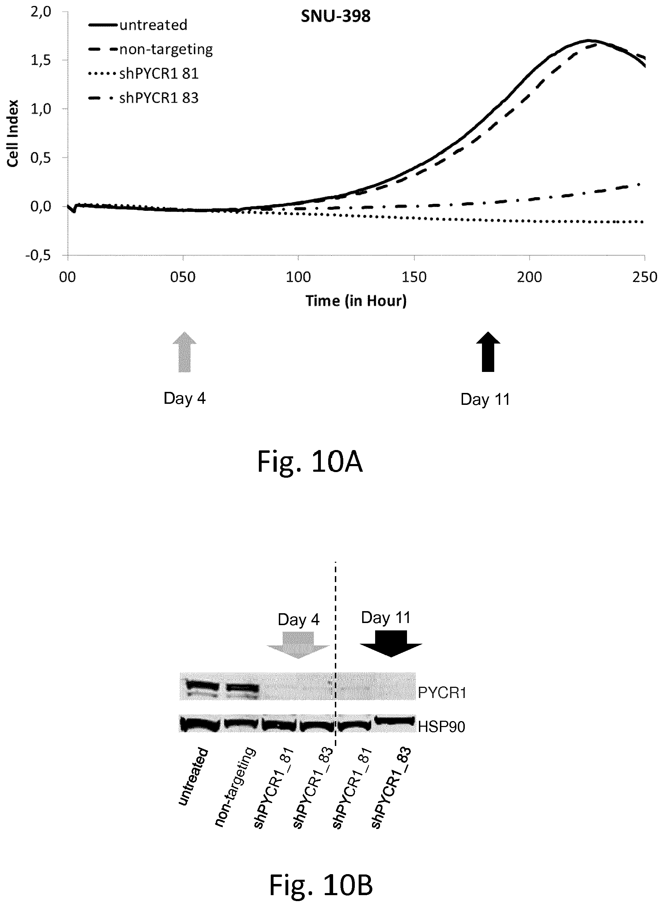

### Example 10: Proliferation assay of cancer cell lines with shRNA-silenced PYCR1 using the xCELLigence system (Figs. 10-13)

Cell culture reagents:
- DMEM/HAM's F12 with L-Glutamine (Gibco #31331-028)
- RPMI-1640 with GlutaMAX (Gibco #61870-010)
- McCoy's 5a (ATCC® 30-2007)
- Minimum Essential Medium (MEM) (Gibco #31095)
- Hams F12 (Biochrom # F 0815)
- Fetal Bovine Serum Superior (Biochrom #S0615)
- L-Alanyl-L-Glutamine (Biochrom # K 0302)
- Natriumpyruvat; (Biochrom # L 0473)
- Non Essentiell Amino Acids; (Biochrom # K 0293)
- Penicillin/Streptomycin (PAA #P11-010)
- PBS 1x (PAA #P15-002)
- TrypLE Express (Gibco, #12-604-013)

### Cell culture media compositions:

| **Cell line** | **Organ** | **Medium** |
|---|---|---|
| SNU-398 | liver | RPMI-1640 + 10% FCS + 1% P/S |
| HepG2 | liver | RPMI-1640 + 10% FCS + 1% P/S |
| Hep3B2 | liver | MEM + 10% FCS + 1% P/S |
| MH3924A (or MH3924a) | liver (rat) | DMEM/ Hams F12 +10% FCS + 1% P/S |
| A375 | skin | DMEM/ Hams F12 +10% FCS + 1% P/S |
| G-361 | skin | McCoy's 5a + 10% FCS + 1% P/S |
| SK-MEL-5 | skin | MEM + 10% FCS + 1% P/S |
| MeWo | skin | MEM + 10% FCS + 1% P/S |
| WM-115 | skin | MEM + 10% FCS + 1% P/S + 2mM L-Alanyl-L-Glutamine |
| KYSE-140 | esophagus | RPMI-1640 + 10% FCS + 1% P/S |
| HaCat | skin | DMEM/ Hams F12 +10% FCS + 1% P/S |

### shRNA's:

- pLKO-TRC1 (non-targeting, Sigma Aldrich)
- pLKO-TRC1 PYCR1-sh81 (TRCN0000038981, Sigma Aldrich) (SEQ ID 14 GAGGGTCTTCACCCACTCCTA)
- pLKO-TRC1 PYCR1-sh83 (TRCN0000038983, Sigma Aldrich) (SEQ ID 15 TGAGAAGAAGCTGTCAGCGTT)

### xCelligence:

- xCELLigence System, RTCA MP Instrument:
   - RTCA MP System Bundle (ACEA Bioscience # 00380601040)
   - E-Plate 96-well (ACEA Bioscience # 05232376001)
   - 2000 cells were seeded per well in a volume of 150µl/well

### Western Blotting:

- RIPA-Lysis buffer:
   50µL per 6well
   - RIPA-Lysis Buffer, 10x (#20-188; Fa. Millipore)
   - Halt Protease and Phosphatase Inhibitor Cocktail (#1861282; Thermo Scientific)
- BCA Protein Assay Reagent (#23225, Thermo Scientific)
- 5% milk powder in 1xTBST
- iBlot 2 Gel Transfer Device (#IB21001, Life Technologies)
- iBlot 2 NC Regular Stacks (#IB23001, Life Technologies)
- Bolt® 4-12% Bis-Tris Gel, 10 Well (#NW04120Box, Life Technologies)
- NuPAGE® Sample Reducing Agent (10X)(# NP0004, Life Technologies)
- NuPAGE® LDS Sample Buffer (4X)(#NP0007, Life Technologies)
- NuPAGE® MOPS SDS Running Buffer (#NP001, Life Technologies)

### Antibodies:

PYCR1 (13108-1-AP, Proteintech Group, used 1:1000)
HSP90 as loading control (610419, BD Bioscience, used 1:5000)
IRDye 800C as secondary antibody (926-32210, LICOR, used 1:10000)
IRDye 680RD as secondary antibody(926-32210, LICOR, used 1:10000)

All cell lines used in the following experiments are available from ATCC (American Type Culture Collection, Manassas, VA 20110-2209). shRNA expressing lentiviruses were produced at the NMI, Kusterdingen, Germany. 1 million cells in 1 ml medium / well were seeded in a 6well plate and 25µl of lentivirus expressing non-targeted or PYCR1 specific shRNAs (listed below) were added 6 hours later. The following day (day 1), the cells were washed with PBS, trypsinated and 1.5-5 million cells were seeded in 2 wells of a 6well culture plate for lysate generation and 2000 cells in 150 µl medium per well were seeded in triplicates in a 96well xCELLigence E-plate to monitor proliferation for about 10 days (OMNI Life Science). Non-treated cells were used as control. On day 3 or 4, the medium was exchanged and 0.6µg/ml puromycin was added to select for shRNA expressing cells. On day 4 and day 8, 9 or 11, lysates were generated from the control and transfected cells grown in 6well plates using 50 µl of RIPA buffer / well, followed by protein determination (Protein Assay Reagent) and 40 µg lysate protein / lane were separated by SDS-PAGE and blotted using the iBlot system.

For PYCR1 knockdown confirmation, the blot membrane was blocked with 2% nonfat milk powder, washed with TBST buffer and incubated with the PYCR1 specific antibody over night at 4°C. After washing 3 times with TBST for 5 min each the blot membrane was incubated for 45 min with the secondary antibody and after washing again 3 times with TBST for 5 min and once with PBS, the blot was analyzed using the Odyssey Imaging System (LI-COR Biotechnology - GmbH).

The human liver carcinoma cell lines SNU-398, HepG2 and Hep3B2 as well as the rat Morris Hepatoma cell line MH3924A and the non-tumorigenic HaCaT cell line were transfected with 2 PYCR1 shRNAs and a non-targeted shRNA as control and proliferation was monitored in comparison to untreated cells using the xCELLigence system (OMNI Life Science). Knockdown was confirmed by Western blot on days 4 and 8 or 11 after transfection. PYCR1 knockdown results in anti-proliferative effects in the liver cancer cell lines while PYCR1 silencing did not significantly inhibit proliferation or cause cell death of non-tumorigenic HaCaT cells. Similar results were obtained for the melanoma cell lines A-375, G-361, SK-MEL-5, MeWo and WM-115 and for the oesophageal cancer cell line KYSE-140.

### Example 11: Determination of cellular proline levels upon PYCR1 shRNA knockdown

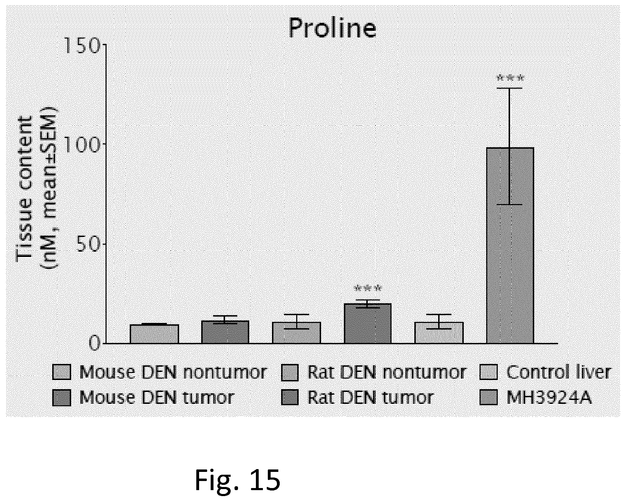

Human hepatocellular carcinoma cell lines SNU-398 and SNU-449 in Minimum Essential Medium (Gibco #31095) supplemented with 10% FBS (Fetal Bovine Serum Superior, Biochrom #S0615) and 1% Penicillin/Streptomycin (PAA #P11-010) were seeded at 1 million cells/ml in 1 ml medium / well in a 6well plate. 25µl of lentivirus expressing non-targeted or PYCR1 specific shRNAs (listed in Example 10) were added 6 hours later. The following day (day 1), the cells were washed with PBS, trypsinated and 250000 cells were seeded in 5 wells of a 6well culture plate for sample generation. Untransfected cells were used as control. On day 5, the medium was exchanged and 0.6µg/ml puromycin was added to select for shRNA expressing cells. On day 6, lysates were generated from one well to confirm PYCR1 knockdown by Western blot and methanolic extracts were generated from 4 wells for LC-MS measurement of proline. Cells were washed twice with cold 0.9% NaCl solution and extracted with2x0.5 ml 80% cold methanol (v/v). After centrifugation, the clear supernatant was used for LC-MS/MS based proline measurement using a Waters ACQUITY UPLC I-Class system connected to a Waters Xevo TQ-S micro triple quadrupole mass spectrometer. The UPLC was equipped with an ACQUITY UPLC BEH Amide 1.7µm 2.1 x 75 mm column and an ACQUITY BEH Amide 1.7µm VANGUARD pre-column. The mass spectrometer was operating in MRM ESI positive mode. Cell pellets were lysed and protein determined (BCA Protein Assay Reagent (#23225, Thermo Scientific) for normalization. PYCR1 knockdown was confirmed and led to reduction of cellular proline levels in both cell lines (Fig. 15). Inhibition of PYCR1 catalytic activity by an antagonist is expected to have the same effect on cellular proline levels.

### Figures

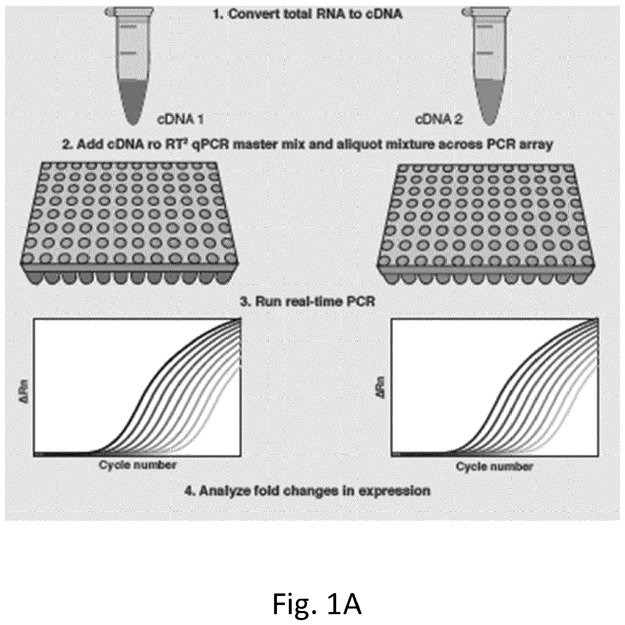

Fig. 1A. PCR array to characterize gene expression in amino acid metabolic pathway. Total RNA from diethylnitrosamine-induced mouse liver tumors and from orthotopic rat MH3924A tumors as well as normal and regenerating liver tissue as controls was applied to the Amino Acid Metabolism II PCR Array for mouse (Qiagen, Cat. No. PAMM-130Z) and rat (Qiagen, Cat. No.PARN-130Z), respectively. Real-time PCR was performed on a StepOnePlus cycler (Applied Biosystems) and data were normalized to the 5 housekeeping genes (ACTB, B2M, GAPDH, HSP90ab1, GUSB) using the ΔΔCT method.
Fig. IB. Up-regulation of the proline biosynthesis pathway in HCC models. Shown is the altered gene expression in DEN-induced mouse tumors and orthotopic rat MH3924A tumors compared to normal liver tissue and regeneration liver tissue. Up-regulated genes are marked by a box with solid lines, down-regulated genes are in *italic* and marked by a box with dashed lines.
Fig. 2: PYCR1 is overexpressed in several tumor entities as shown by Array Northern. The gene probe 202148_s_at was used to probe PYCR1 expression in normal and diseased tissues using the Array Northern application as described in Example 2. Shown are PYCR1 expression data for tumor tissues that display higher PYCR1 expression compared to the corresponding normal tissue.
Fig. 3: Up-regulation of PYCR1 and down-regulation of PRODH in liver tumor tissues. 4 liver tumors of the rat orthotopic MH3924A model and the mouse DEN-induced HCC model as well as 4 normal rat and murine liver samples were analyzed for mRNA (A) and protein (B) expression of PYCR1 as well as protein expression of PRODH (C). The experiments show up-regulation of PYCR1 and lack of PRODH expression in tumors of HCC models compared to control liver tissue.
Fig. 4: PYCR1 expression in human and rat liver tumor cell lines and hepatocytes. (A) PYCR1 expression in human liver tumor cell lines as shown by Array Northern. (B) PYCR1 protein levels in human and rat tumor cells compared to hepatocytes. PYCR1 expression is low in rat and human hepatocytes and increased in rat and human liver tumor cells. PYCR1 protein levels match mRNA data in human HCC cell lines.
Fig. 5: PYCR1 Knockdown in Rat MH3924A cells using 3 different shRNAs. Significant PYCR1 shRNA knock-down on mRNA (A) and protein level (B) reduces cell proliferation (C) of rat Morris Hepatoma cells ((C): left column: day 0, right column: day 4).
Fig. 6: *In vitro* and *in vivo* effects of stable PYCR1 shRNA knockdown in rat MH3924A cells. (A) MH3924A cells stably transfected with PYCR1 shRNA 1.2 (see Fig. 5) showed reduced proliferation and altered morphology compared to MH3924A cells transfected with scrambled shRNA (left column: shRNA scrambled, right column: shRNA PYCR1). (B) MH3924A cells stably expressing PYCR1 shRNA 1.2 were subcutaneously injected into *scid* mice and tumors excised 3 weeks later. PYCR1 shRNA knockdown significantly inhibited tumor growth *in vivo* as shown by reduced tumor weight and barely visible tumor compared to control in the examplary photograph.
Fig. 7: Effects of inducible PYCR1 shRNA knockdown *in vitro* and *in vivo* in the preventive setting. (A) PYCR1 shRNA 1.2 was used to generate a doxycycline-inducible PYCR1 knockdown MH3924A cell line for *in vivo* studies. Western blot shows reduced expression of PYCR1 upon doxycycline treatment, leading to reduced cell growth (left column: without doxycycline, right column: with doxycycline). (B) One day after orthotopically implanting the PYCR1 knockdown MH3924A cell line into *scid* mice, PYCR1 knockdown was induced by doxycycline and significantly reduced tumor growth in this preventive setting. This provides evidence that the tumor microenvironment does not counteract PYCR1 knockdown effect on tumor cell proliferation.
Fig. 8: PYCR1 knockdown effect on established tumors - 1st experiment. (A) 7 days after orthotopically implanting PYCR1 shRNA expressing MH3924A cells into *scid* mice, PYCR1 knockdown was induced with doxycycline. (B) 11 days later the tumors were analyzed by MRI and tumors excised for analysis. Western blot of tumor lysates shows very weak PYCR1 knockdown (A). There is no significant *in vivo* tumor growth effect in this experiment with a very weak PYCR1 knockdown.
Fig. 9: PYCR1 knockdown effect on established tumors - 2nd experiment. (A) 14 days after orthotopically implanting PYCR1 shRNA MH3924A cells into *scid* mice PYCR1 knockdown was induced with doxycycline. (B) 7 days later the tumors were analyzed by MRI and tumors excised for analysis. There is a trend to slowed tumor growth in the therapeutic setting with weak PYCR1 knockdown.
Fig. 10: Proliferation of selected tumor cell lines after PYCR1 knockdown. Example shown for cell line SNU-398 (human liver carcinoma cell line). Cells were transfected with 2 PYCR1 shRNAs and a non-targeted shRNA as control and proliferation monitored in comparison to untreated cells using the xCELLigence system (OMNI Life Science) (A). Knockdown was confirmed by Western blot on days 4 and 11 after transfection (B). Similar results were obtained for

| | |
|---|---|
| HepG2 | Human hepatocellular carcinoma |
| Hep3B2 | Human hepatoma |
| MH3924A | Rat hepatoma |
| A-375 | Human melanoma |
| G-361 | Human amelanotic melanoma |
| SK-MEL-5 | Human melanoma |
| MeWo | Human melanoma |
| WM-115 | Human melanoma |
| KYSE-140 | Esophagus carcinoma |

PYCR1 knockdown results in anti-proliferative effects in these cancer cell lines.

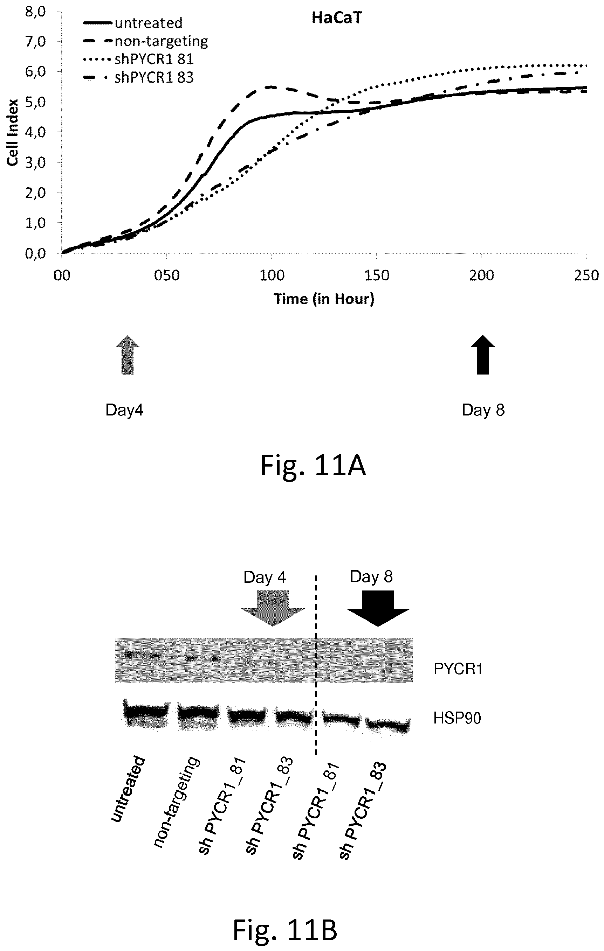

Fig. 11: Proliferation of non-tumorigenic HaCaT cell line after PYCR1 knockdown. PYCR1 was silenced and knockdown and proliferation monitored as described in the legend to Fig. 10. Knockdown does not cause cell death or significantly inhibits proliferation of non-tumorigenic "normal" HaCaT cells (A). Confirmation of knockdown (B).

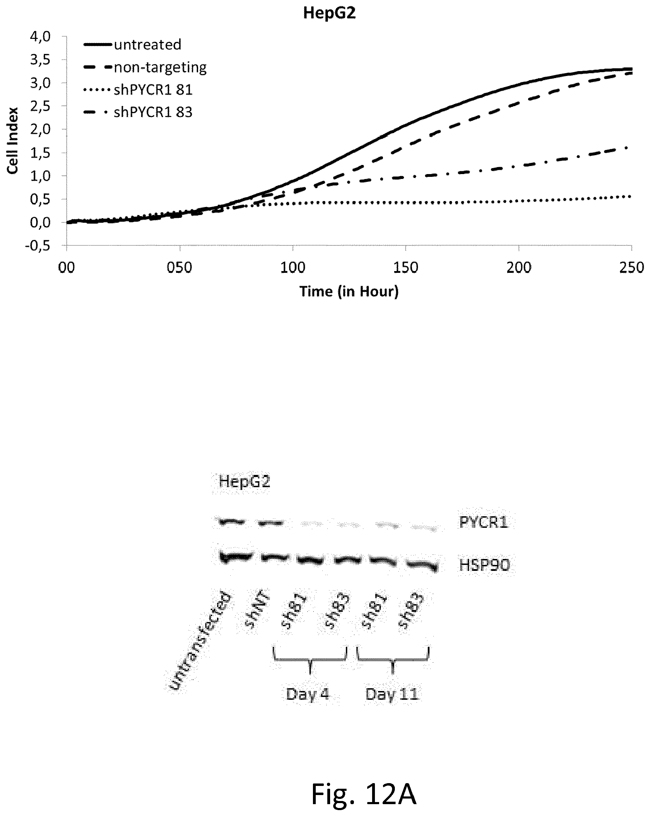

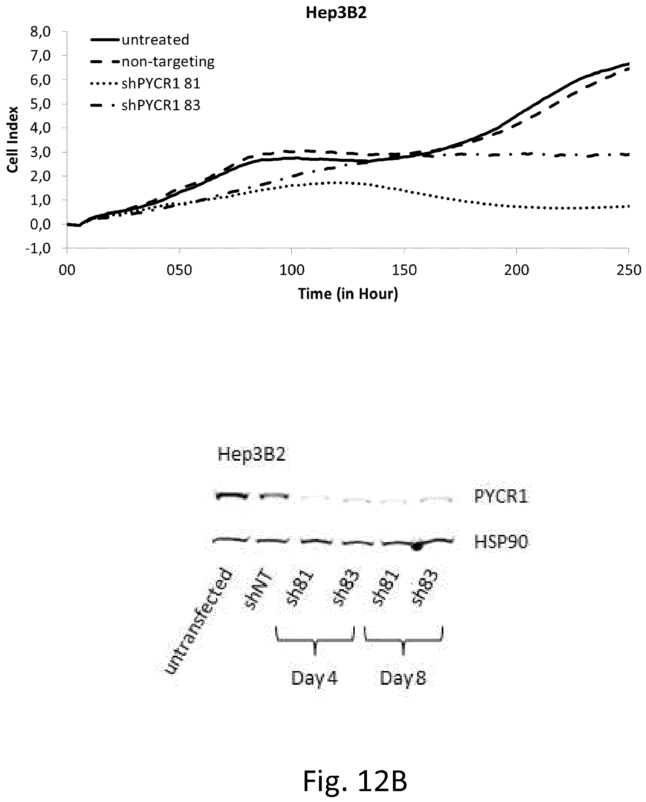

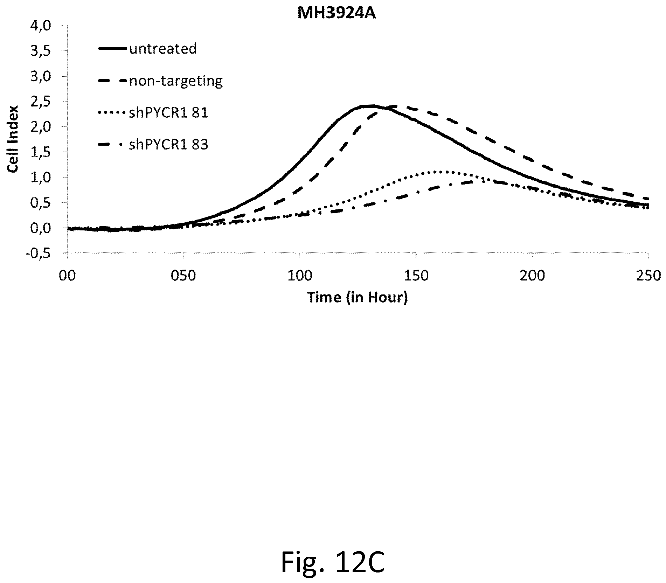

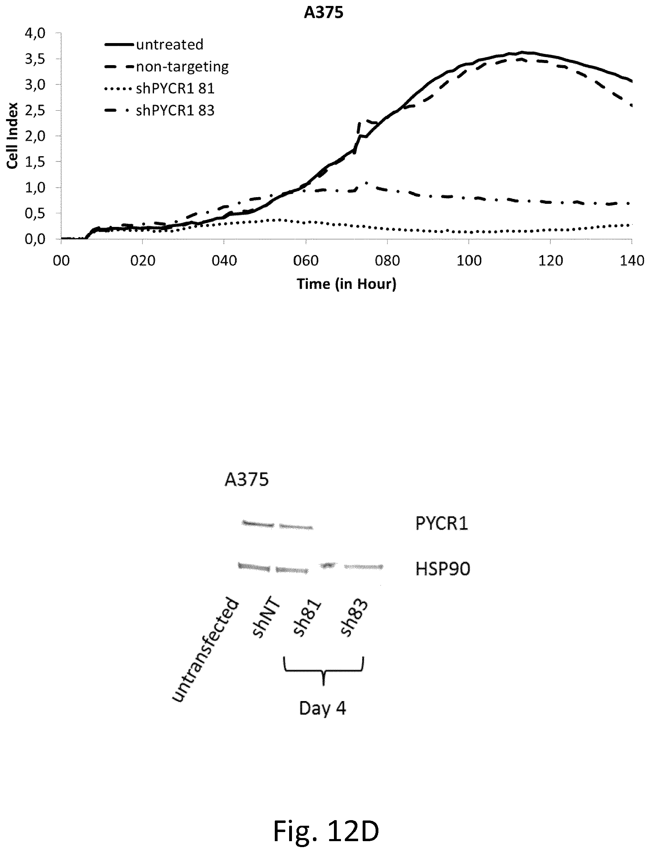

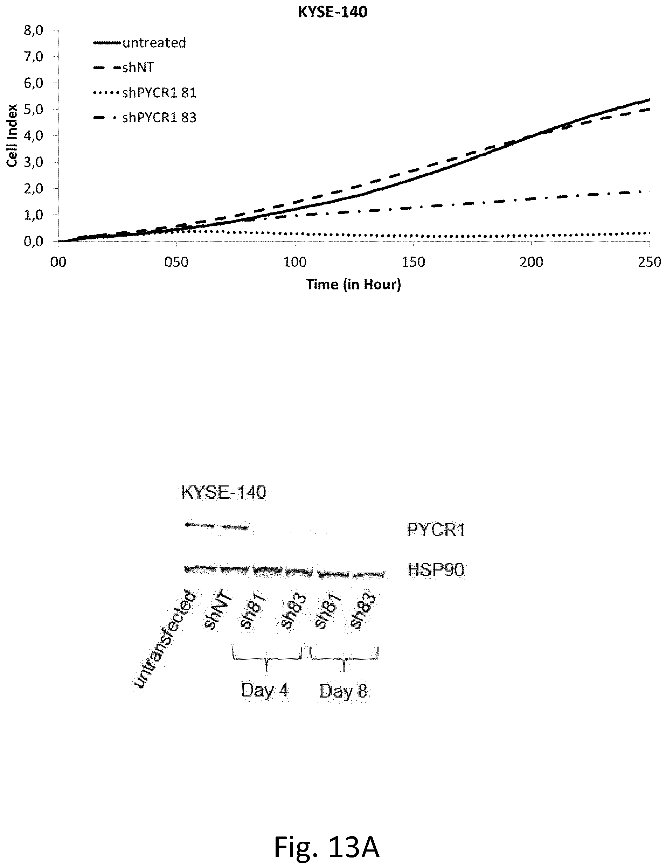

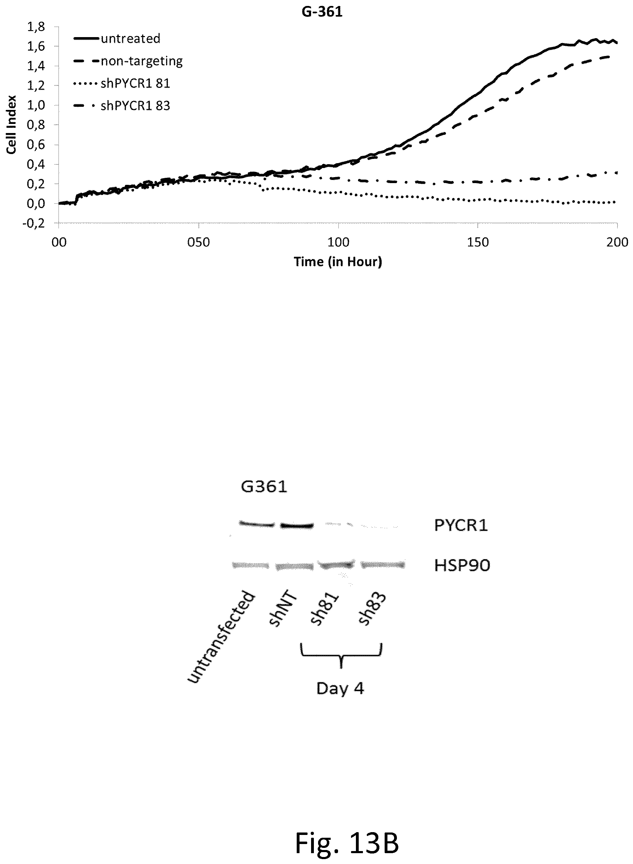

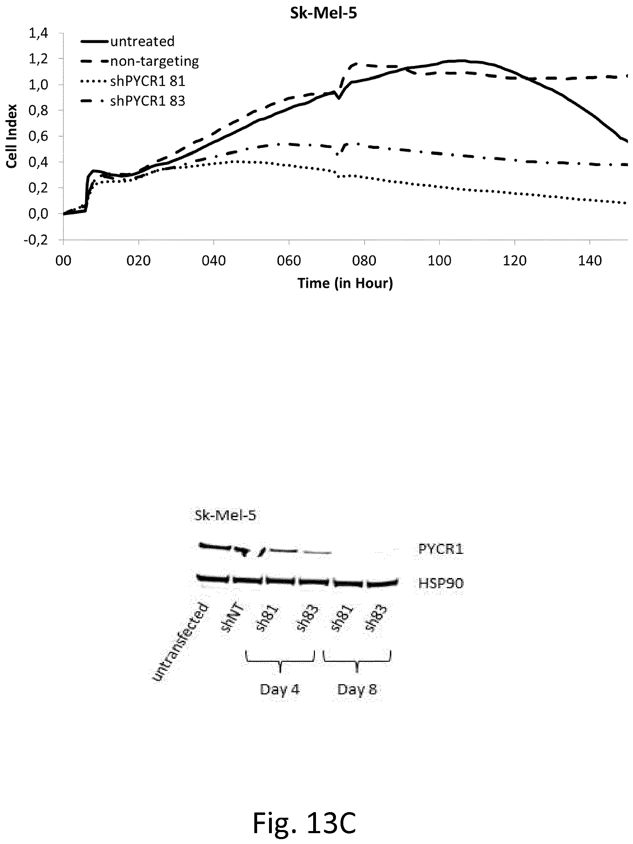

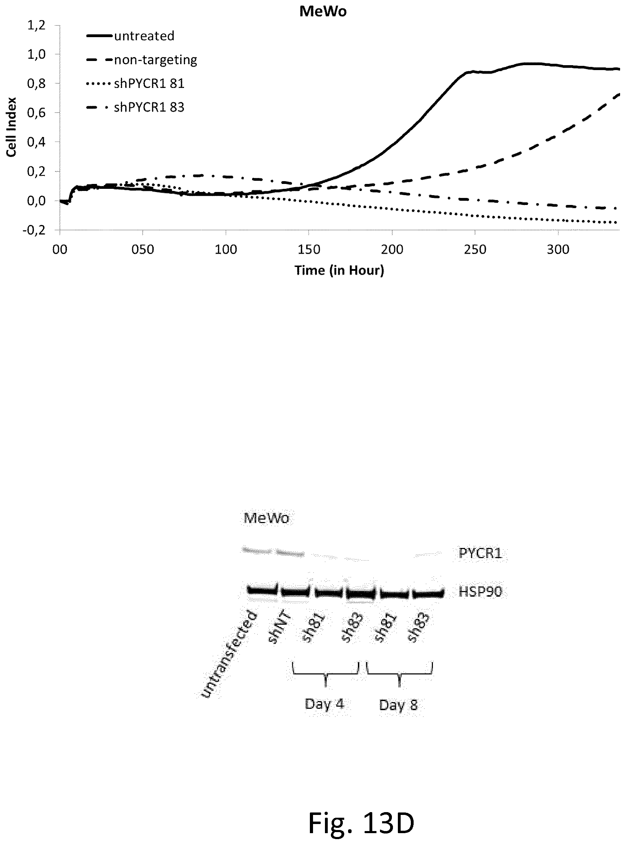

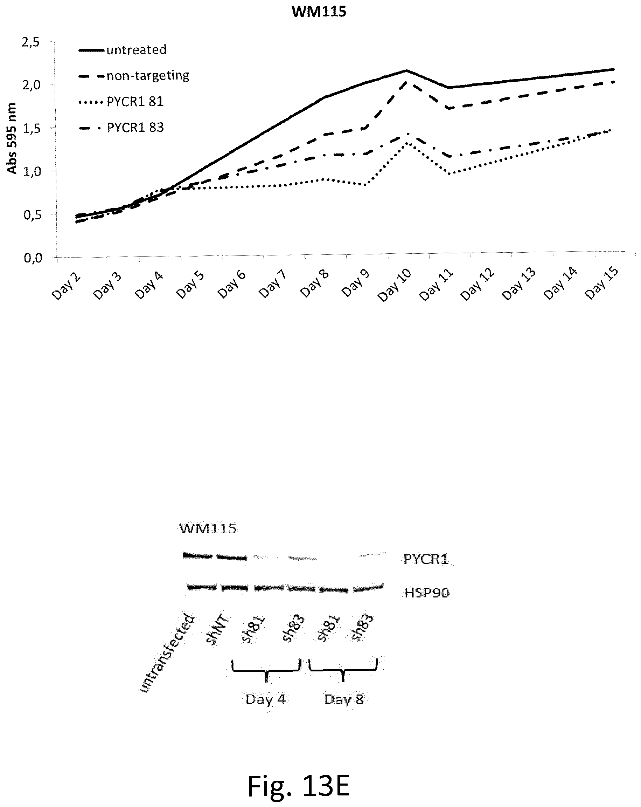

Fig. 12 and 13: Growth inhibition of selected tumor cell lines after PYCR1 knockdown. PYCR1 was silenced and knockdown and proliferation monitored as described in the legend to Fig. 10. Fig. 12: HepG2 (A), Hep3B2 (B), MH3924A (C) and A-375 (D). Fig. 13: KYSE-140 (A) G-361 (B), SK-MEL-5 (C), MeWo (D) and WM-115 (E).

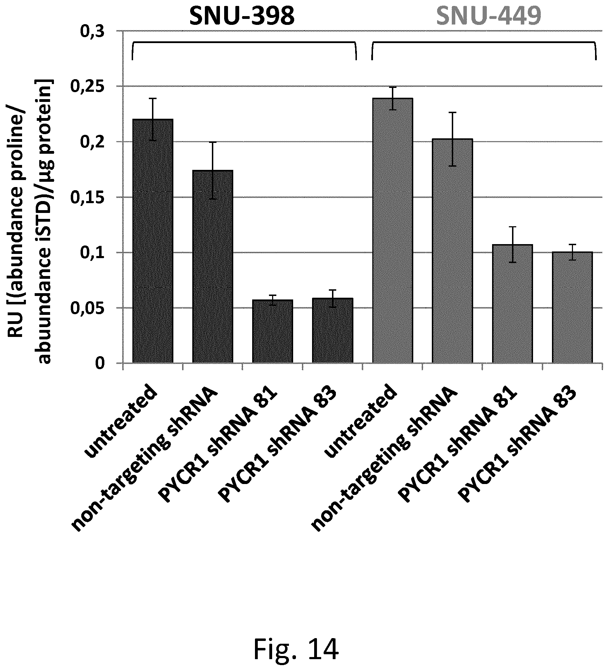

Fig. 14: Determination of cellular proline levels in PYCR1 knockdown cancer cells (SNU-398, SNU-449) by LC-MS. The data show that knockdown of PYCR1 leads to reduction of cellular proline levels in the two cell lines tested.
Fig. 15: Tissue concentration of proline in DEN-induced mouse and rat, and orthotopic MH3924A rat HCC models as determined by mass spectrometry. Statistical analysis was performed using Student's *t*-test. *, P < 0.05. Increased proline levels in tumor versus nontumor tissue are in agreement with increased levels of proline biosynthetic enzymes PYCR1 and P5CS in tumors.

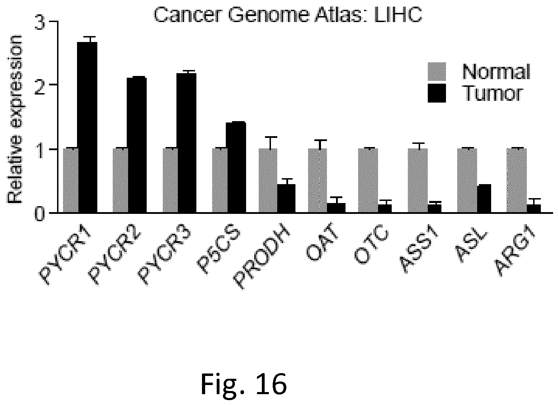

Fig. 16: Proline metabolic enzyme expression is altered in human HCC. Relative expression of genes encoding relevant proline metabolic enzymes in primary HCC tumors and normal liver tissues in The Cancer Genome Atlas (TCGA) cohort. Values are normalized to normal liver tissues. The data show that also in human HCC expression of proline biosynthesis enzymes PYCR1 and also PYCR2 and PYCR3 and P5CS is increased compared to normal liver while expression of catabolic enzymes is decreased. ARG, Arginase; ASS, argininosuccinate synthetase; ASL, argininosuccinate lyase; OAT, ornithine aminotransferase; OTC, ornithine transcarbamylase; PYCR, pyrroline 5-carboxylate reductase; P5CS, pyrroline-5-carboxylate synthase; PRODH, proline dehydrogenase.

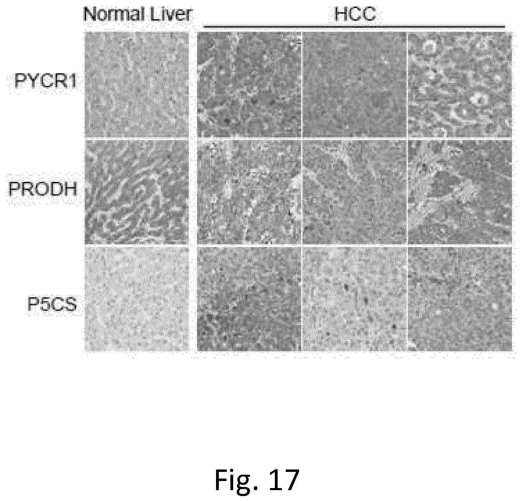

Fig. 17: Representative immunohistochemical images of PYCR1, PRODH, and P5CS expression in human HCC tumors and normal liver tissues, as profiled by The Human Protein Atlas. The data show that PYCR1 and P5CS levels are increased while PRODH levels are decreased in human HCC compared to normal liver.

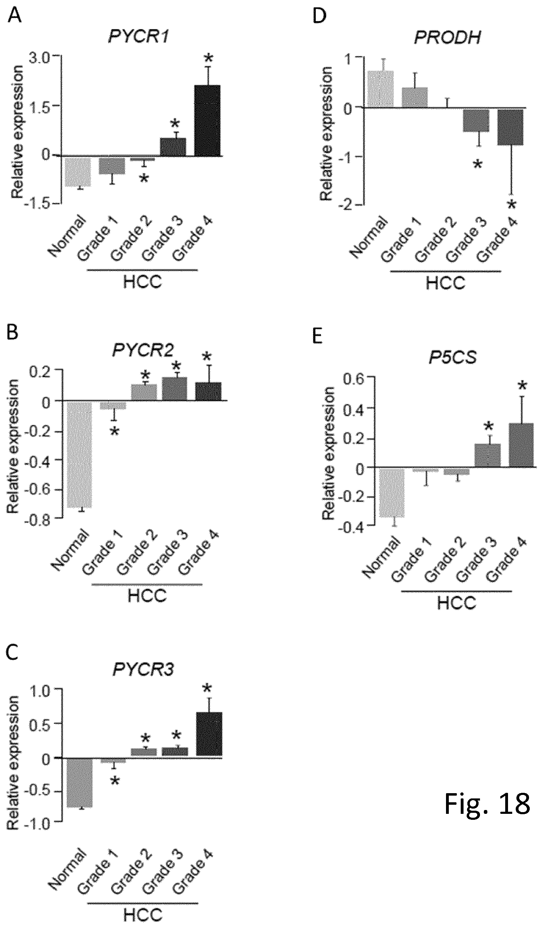

Fig. 18: Relative expression of *PYCR1* (A), *PYCR2* (B), *PYCR3* (C), *PRODH* (D) and *P5CS* (E) genes in human HCC tumors of different grades (TCGA cohort). Bar graphs are shown as mean ± SEM. Statistical analysis was performed using Student's *t*-test. *, P < 0.05. The data show that *PYCR1* and *P5CS* gene expression correlate with HCC grade while *PRODH* gene expression inversely correlates with HCC grade.

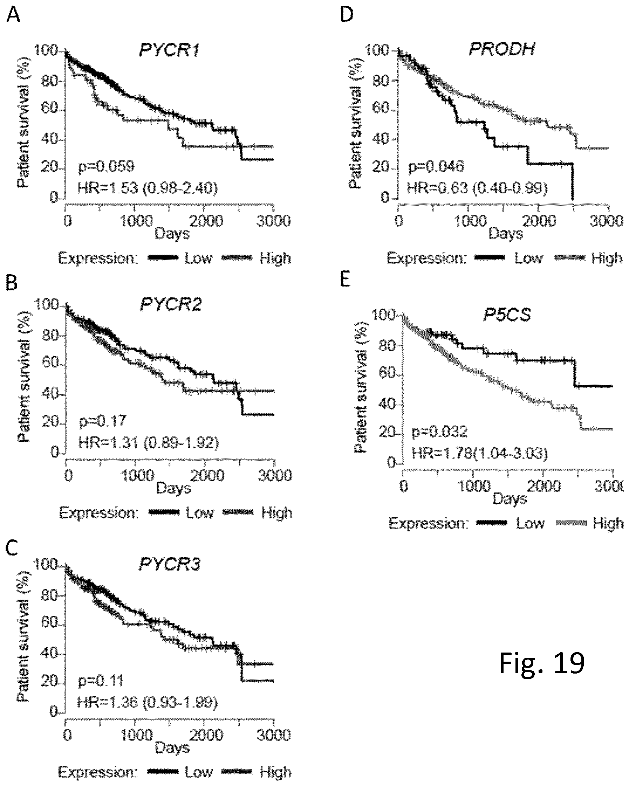

Fig.19: Kaplan-Meier survival estimate curves for HCC patients from the TCGA cohort stratified and grouped by expression levels of the proline metabolic enzymes PYCR1 (A), PYCR2 (B), PYCR3 (C), PRODH (D), and P5CS (E). Log-rank test P-values and Cox proportional hazard ratios for the high expression groups are shown in the graphs. High *PYCR1, PYCR2, PYCR3* or *P5CS* and low *PRODH* tumor expression are independently associated with poor patient prognosis.

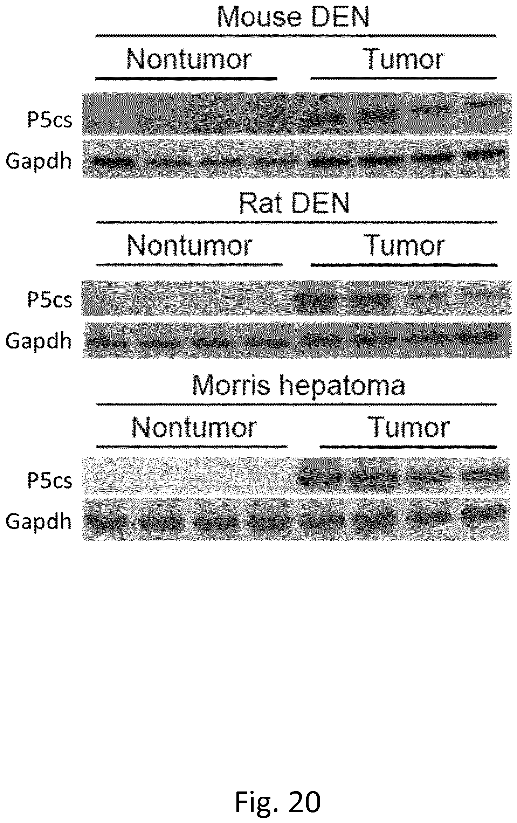

Fig. 20: Pyrroline 5-carboxylate synthase (P5cs) expression in DEN-induced mouse and rat, and orthotopic MH3924A rat HCC tumors as determined by Western blotting. The proline biosynthetic enzyme P5cs is upregulated in liver tumors compared to non-tumor liver tissue.

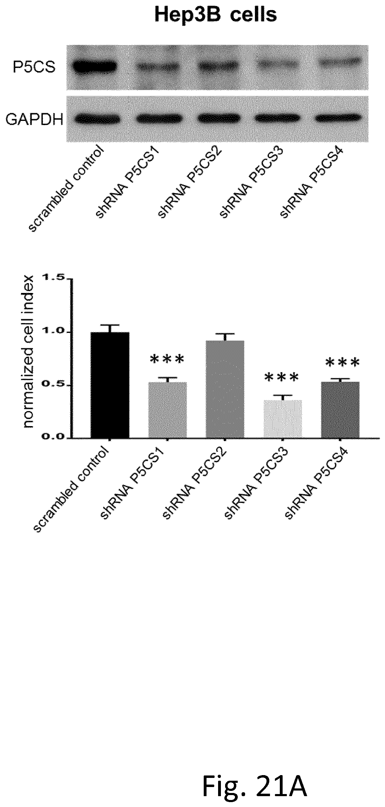

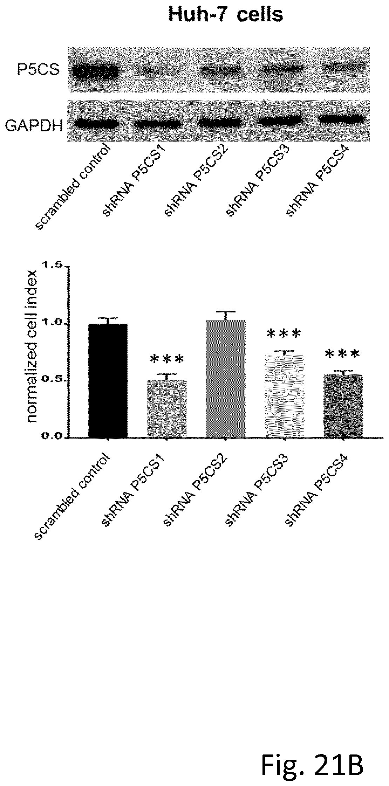

Fig. 21: P5CS expression as determined by Western blotting (top panel) and inhibition of cell proliferation as determined by automated cell counting (lower panel) in Hep3B (A) and Huh7 (B) HCC cells six days after lentiviral P5CS knockdown infection. Statistical analysis was performed using Student's t-test. ***, P < 0.001. P5CS, pyrroline 5-carboxylate synthase.

### Further discussion

The findings made herein do also translate to antagonists of PYCR2 (Pyrroline-5-carboxylate reductase 2, UniProtKB - Q96C36 (P5CR2_HUMAN) and PYCR3 (Pyrroline-5-carboxylate reductase 3, UniProtKB - Q53H96 (P5CR3_HUMAN)).

PYCR1 shows high sequence similarity/identity not only in its isoforms, but also with PYCR2 and PYCR3. The active sites of all three variants are well conserved, showing 100 % identity in its isoforms, but still 97.1 % identity between PYCR1 and PYCR2, and 85,7 % similarity/60% identity between PYCR1 and PYCR3. The next-closest relative is NXRD1 (NADP-dependent oxidoreductase domain-containing protein 1), which features 32% overall identity with PYCR1 but hardly any identity/similarity in the active site.

Further, because of the sequence similarity, an involvement of PYCR2 and PYCR3 in oncogenesis, in particular of liver tumor, skin tumor and oesophagus tumor, is highly likely.

Hence, inhibitors against the active site of PYCR1, including antibodies, aptamers and small molecules, are expected to also inhibit PYCR2, while inhibition of PYCR3 appears likely at least in many cases. These inhibitors are hence likely to also exhibit an antineoplastic effect, in particular in the treatment of liver tumor, skin tumor and oesophagus tumor. Similarly, nucleic acid molecules that bind to PYCR1 encoding nucleic acid may also inhibit PYCR2 and inhibition of PYCR3 appears possible.

Further, the assays disclosed herein can likewise be used to actually identify inhibitors for PYCR2 and PYCR3, e.g., by
(i) replacing PYCR1 as an enzyme (e.g., in an inhibition assay)
(ii) replacing PYCR1 as a target antigen (e.g., in a display approach, or in aptamer screening)
(iii) replacing the PYCR1 target sequence (e.g., when creating an inhibitory nucleic acid).

Furthermore, proline metabolism was found to be altered in human HCC and associated with patient prognosis:
The gene expression changes found in the HCC animal models were found to be similar to changes seen in human HCC tumors in the TCGA RNA-seq data. Potent upregulation of PYCR1 and P5CS and downregulation of PRODH were observed in both analyses (Fig. 1B, 16). Representative immunohistochemical images from The Human Protein Atlas for PYCR1, PRODH, and P5CS in HCC tumors and normal liver tissues confirmed the expression changes on the protein level (Fig. 17). Increased expression of the three PYCR isoforms PYCR1, PYCR2 and PYCR3, and of P5CS and decreased expression of PRODH were associated with increasing HCC tumor grades, suggesting that the proline metabolic pathway may be particularly altered in poorly-differentiated, aggressive tumors (Fig. 18).

Clinical data demonstrated that patients who retained high tumor expression of PRODH, or low tumor expression of PYCR1 (and also PYCR2 and PYCR3) or P5CS had significantly better survival rates, as determined by analysis of the TGCA cohort for correlations in overall survival (Fig. 19).

### References

Reversade et al (2009), Nature Genetics 41, 1016 - 1021
Kretz et al (2011), Journal of Inherited Metabolic Disease 34 (3), 731-739
Roth et al, Proteomics, 10: 194-202.
Köhler & Milstein (1975), Nature 256, 495-497
Chen & Erlanger (2002), Immunol Lett. 21;84(1):63-7
Berguig et al (2015) Mol Ther 23(5):907-17
Meunier, Executive Interview - BioCellChallenge: Optimizing the Potential of Intracellular
Thura et al (2016) JCI Insight 1(9):e87607
Therapeutic Antibodies Drug Dev. April 2014, Posted Date: 3/31/2014.
Xu & Wang (2015), Asian Journal of Pharmaceutical Sciences 10 (1), 1-12.
Jinek et al (2012), Science 17;337(6096): 816-21
Yin et al (2016), Nature Biotechnology 34,328-333
Zetsche et al (2015), Cell 22;163(3):759-71
Blind & Blank (2015), Molecular Therapy Nucleic Acids (2015) 4, e223.
Thiel & Giangrande (2010), Ther Deliv. 1(6):849-61.
Verdine and Hilinski (2012), Methods in enzymology. 503: 3-33
Walensky and Bird, (2014). Journal of Medicinal Chemistry. 57 (15): 6275-88
Mitchell and Willenbring, Nature Protocols 3, - 1167 - 1170 (2008)
Boroughs and DeBerardinis, Nat Cell Biol., 2015 Apr;17(4):351-9
Locasale, Grassian et al, Nat Genet. 2011 Jul 31;43(9):869-74.
Possemato, Marks et al., Nature. 2011 Aug 18;476(7360):346-50
El-Serag, N Engl J Med. 2011 Sep 22;365(12): 1118-27
Llovet, Ricci et al., N Engl J Med 2008; 359:378-390

## Claims

1. An antagonist of PYCR1 for the treatment and/or prevention of a neoplastic disease.

2. The antagonist of claim 1, wherein the neoplastic disease is a liver tumor, or a secondary tumor derived from a liver tumor.

3. The antagonist of claim 1, wherein the neoplastic disease is a skin tumor, or a secondary tumor derived from a skin tumor.

4. The antagonist of claim 1, wherein the neoplastic disease is an oesophageal tumor, or a secondary tumor derived from an oesophageal tumor.

5. The antagonist according to any of the above mentioned claims, which inhibits, directly or indirectly, PYCR1-mediated formation of L-Proline.

6. The antagonist according to any of the above mentioned claims, which is a monoclonal antibody, or a target-binding fragment or derivative thereof retaining target binding capacities, that specifically binds to one or more isoforms of the PYCR1 protein.

7. The antagonist according to any of claims 1 - 5, which antagonist comprises a first nucleic acid molecule that specifically binds to a second nucleic acid molecule, which second nucleic acid molecule encodes an isoform of the PYCR1 protein.

8. The antagonist according to any of claims 1 - 5, which is an aptamer that specifically binds to one or more isoforms of the PYCR1 protein.

9. The antagonist according to any of claims 1 - 5, which is a small molecule that specifically binds to to one or more isoforms of the PYCR1 protein.

10. The antagonist according to any of the above mentioned claims, which antagonist can be found by means of a PYCR1 inhibition assay, where the impact of a candidate molecule on the PYCR1-catalyzed transformation from
• L-Proline to L-1 pyrroline-5-carboxylate, or
• L-1 pyrroline-5-carboxylate to L-Proline
is determined.

11. The antagonist according to any of claims 6, 8 or 9, wherein the PYCR1 protein to which the antibody, aptamer or small molecule binds comprises a consensus sequence according to SEQ ID No 10.

12. The antagonist according to any of claims 6, 8 or 9, wherein the PYCR1 protein to which the antibody, aptamer or small molecule binds comprises a sequence according to any of SEQ ID Nos 6 - 8.

13. The antagonist according to claim 7, wherein the nucleic acid encoding an isoform of the PYCR1 protein comprises a consensus sequence according to SEQ ID No 9.

14. The antagonist according to claim 7 or 13, wherein the nucleic acid encoding an isoform of the PYCR1 protein comprises a sequence according to any of SEQ ID Nos 1 - 5.

15. The antagonist according to any of the aforementioned claims, wherein the neoplastic disease is **characterized by** overexpression of the PYCR1 gene and/or reduced expression of the ProDH gene.

16. The antagonist according to any of the aforementioned claims wherein the neoplastic disease is **characterized by** an excess of the PYCR1 protein.

17. Use of the antagonist according to any of the aforementioned claims (for the manufacture of a medicament) in the treatment of a human or animal subject being diagnosed for, suffering from or being at risk of developing a neoplastic disease, or for the prevention of such condition.

18. A pharmaceutical composition comprising an antagonist according to any of claims 1 - 16.

19. A combination of a pharmaceutical composition according to claim 18 and one or more further therapeutically active compounds.

20. A method for treating or preventing a disorder or condition associated with the undesired expression of PYCR1, comprising administering to a subject in need thereof an effective amount of an antagonist according to any of the claims 1 to 16, a pharmaceutical composition according to claim 17 or a combination according to claim 19.


SEQUENCE LISTING

<110> Bayer Pharma Aktiengesellchaft
      Agency for Science, Technology and Research

<120> Inhibitors and antagonists of human PYCR1

<130> BHC163058 FC

<160> 13

<170> BiSSAP 1.3.6

<210> 1
<211> 1811
<212> DNA
<213> NM_001282279.1: Homo sapiens pyrroline-5-carboxylate reductase 1
(PYCR1), transcript variant 3, mRNA.


<400> 1
ggattcgctg gcgcgacggg ggcggggcct gggtgcgggt gccgcgtggc ggccgcagcg      60

ctgagggagc cggtgccatc tgtgggggct ttgggccagg ggtctccgga cagcatgagc     120

gtgggcttca tcggcgctgg ccagctggct tttgccctgg ccaagggctt cacagcagca     180

ggcgtcttgg ctgcccacaa gataatggct agctccccag acatggacct ggccacagtt     240

tctgctctca ggaagatggg ggtgaagttg acaccccaca acaaggagac ggtgcagcac     300

agtgatgtgc tcttcctggc tgtgaagcca cacatcatcc ccttcatcct ggatgaaata     360

ggcgccgaca ttgaggacag acacattgtg gtgtcctgcg cggccggcgt caccatcagc     420

tccattgaga agaagctgtc agcgtttcgg ccagccccca gggtcatccg ctgcatgacc     480

aacactccag tcgtggtgcg ggagggggcc accgtgtatg ccacaggcac gcacgcccag     540

gtggaggacg ggaggctcat ggagcagctg ctgagcagcg tgggcttctg cacggaggtg     600

gaagaggacc tgattgatgc cgtcacgggg ctcagtggca gcggccccgc ctacggggct     660

gccaagatgc tgctgcactc agaacagcac ccaggccagc tcaaggacaa cgtcagctct     720

cctggtgggg ccaccatcca tgccttgcat gtgctggaga gtgggggctt ccgctccctg     780

ctcatcaacg ctgtggaggc ctcctgcatc cgcacacggg agctgcagtc catggctgac     840

caggagcagg tgtcaccagc cgccatcaag aagaccatcc tggacaaggt gaagctggac     900

tcccctgcag ggaccgctct gtcgccttct ggccacacca agctgctccc ccgcagcctg     960

gccccagcgg gcaaggattg acacgtcctg cctgaccacc atcctgccac caccttctct    1020

tctcttgtca ctagggggac tagggggtcc ccaaagtggc ccactttctg tggctctgat    1080

cagcgcaggg gccagccagg gacatagcca gggaggggcc acatcacttc ccactggaaa    1140

tctctgtggt ctgcaagtgc ttcccagccc agaacagggg tggattcccc aacctcaacc    1200

tcctttcttc tctgctccca aaccatgtca ggaccacctt cctctagagc tcgggagccc    1260

ggagggtctt cacccactcc tactccagta tcagctggca cgggctcctt cctgagagca 1320

aaggtcaagg accccctctg tgaaggctca gcagaggtgg gatcccacgc cccctcccgg 1380

cccctccctg ccctccattc agggagaaac ctctccttcc cgtgtgagaa gggccagagg 1440

gtccaggcat cccaagtcca gcgtgaaggg ccacagcccc tcttggctgc caagcacgca 1500

gatcccatgg acatttgggg aaagggctcc ttgggctgct ggtgaacttc tgtggccacc 1560

acctcctgct cctgacctcc ctgggagggt gctatcagtt ctgtcctggc cctttcagtt 1620

ttataagttg gtttccagcc cccagtgtcc tgacttctgt ctgccacatg aggagggagg 1680

ccctgcctgt gtgggagggt ggttactgtg ggtggaatag tggaggcctt caactgatta 1740

gacaaggccc gcccacatct tggagggcat ctgccttact gattaaaatg tcaatgtaat 1800

ctaatgcaaa a 1811

<210> 2
<211> 1904
<212> DNA
<213> NM_001282280.1 Homo sapiens pyrroline-5-carboxylate reductase 1
(PYCR1),

<400> 2
ggattcgctg gcgcgacggg ggcggggcct gggtgcgggt gccgcgtggc ggccgcagcg 60

ctgagggagc cggtgccatc tgtgggggct ttgggccagg ggtctccgga cagcatgagc 120

gtgggcttca tcggcgctgg ccagctggct tttgccctgg ccaagggctt cacagcagca 180

ggcgtcttgg ctgcccacaa gataatggct agctccccag acatggacct ggccacagtt 240

tctgctctca ggaagatggg ggtgaagttg acaccccaca acaaggagac ggtgcagcac 300

agtgatgtgc tcttcctggc tgtgaagcca cacatcatcc ccttcatcct ggatgaaata 360

ggcgccgaca ttgaggacag acacattgtg gtgtcctgcg cggccggcgt caccatcagc 420

tccattgaga agaagctgtc agcgtttcgg ccagccccca gggtcatccg ctgcatgacc 480

aacactccag tcgtggtgcg ggagggggcc accgtgtatg ccacaggcac gcacgcccag 540

gtggaggacg ggaggctcat ggagcagctg ctgagcagcg tgggcttctg cacggaggtg 600

gaagaggacc tgattgatgc cgtcacgggg ctcagtggca gcggccccgc ctacgcattc 660

acagccctgg atgccctggc tgatgggggt gtgaagatgg gacttccaag gcgcctggca 720

gtccgcctcg gggcccaggc cctcctgggg gctgccaaga tgctgctgca ctcagaacag 780

cacccaggcc agctcaagga caacgtcagc tctcctggtg gggccaccat ccatgccttg 840

catgtgctgg agagtggggg cttccgctcc ctgctcatca acgctgtgga ggcctcctgc 900

atccgcacac gggagctgca gtccatggct gaccaggagc aggtgtcacc agccgccatc 960

aagaagacca tcctggacaa ggtgaagctg gactcccctg cagggaccgc tctgtcgcct 1020

tctggccaca ccaagctgct cccccgcagc ctggccccag cgggcaagga ttgacacgtc 1080

ctgcctgacc accatcctgc caccaccttc tcttctcttg tcactagggg gactaggggg 1140

tccccaaagt ggcccacttt ctgtggctct gatcagcgca ggggccagcc agggacatag 1200

ccagggaggg gccacatcac ttcccactgg aaatctctgt ggtctgcaag tgcttcccag 1260

cccagaacag gggtggattc cccaacctca acctcctttc ttctctgctc ccaaaccatg 1320

tcaggaccac cttcctctag agctcgggag cccggagggt cttcacccac tcctactcca 1380

gtatcagctg gcacgggctc cttcctgaga gcaaaggtca aggaccccct ctgtgaaggc 1440

tcagcagagg tgggatccca cgccccctcc cggcccctcc ctgccctcca ttcagggaga 1500

aacctctcct tcccgtgtga gaagggccag agggtccagg catcccaagt ccagcgtgaa 1560

gggccacagc ccctcttggc tgccaagcac gcagatccca tggacatttg gggaaagggc 1620

tccttgggct gctggtgaac ttctgtggcc accacctcct gctcctgacc tccctgggag 1680

ggtgctatca gttctgtcct ggccctttca gttttataag ttggtttcca gcccccagtg 1740

tcctgacttc tgtctgccac atgaggaggg aggccctgcc tgtgtgggag ggtggttact 1800

gtgggtggaa tagtggaggc cttcaactga ttagacaagg cccgcccaca tcttggaggg 1860

catctgcctt actgattaaa atgtcaatgt aatctaatgc aaaa 1904

<210> 3
<211> 1897
<212> DNA
<213> NM_001282281.1 Homo sapiens pyrroline-5-carboxylate reductase 1
(PYCR1),transcript variant 5, mRNA.

<400> 3
agctgggttg gggtcgggct ccgggaatgg tgggcggcgg tcgccgggtg gggcgcgacg 60

agccggtgcc atctgtgggg gctttgggcc aggggtctcc ggacagcatg agcgtgggct 120

tcatcggcgc tggccagctg gcttttgccc tggccaaggg cttcacagca gcaggcgtct 180

tggctgccca caagataatg gctagctccc cagacatgga cctggccaca gtttctgctc 240

tcaggaagat gggggtgaag ttgacacccc acaacaagga gacggtgcag cacagtgatg 300

tgctcttcct ggctgtgaag ccacacatca tccccttcat cctggatgaa ataggcgccg 360

acattgagga cagacacatt gtggtgtcct gcgcggccgg cgtcaccatc agctccattg 420

agaagaagct gtcagcgttt cggccagccc ccagggtcat ccgctgcatg accaacactc 480

cagtcgtggt gcgggagggg gccaccgtgt atgccacagg cacgcacgcc caggtggagg 540

acgggaggct catggagcag ctgctgagca gcgtgggctt ctgcacggag gtggaagagg 600

acctgattga tgccgtcacg gggctcagtg gcagcggccc cgcctacgca ttcacagccc 660

tggatgccct ggctgatggg ggtgtgaaga tgggacttcc aaggcgcctg gcagtccgcc 720

tcggggccca ggccctcctg ggggctgcca agatgctgct gcactcagaa cagcacccag 780

gccagctcaa ggacaacgtc agctctcctg gtggggccac catccatgcc ttgcatgtgc 840

tggagagtgg gggcttccgc tccctgctca tcaacgctgt ggaggcctcc tgcatccgca 900

cacgggagct gcagtccatg gctgaccagg agcaggtgtc accagccgcc atcaagaaga 960

ccatcctgga caaggtgaag ctggactccc ctgcagggac cgctctgtcg ccttctggcc 1020

acaccaagct gctcccccgc agcctggccc cagcgggcaa ggattgacac gtcctgcctg 1080

accaccatcc tgccaccacc ttctcttctc ttgtcactag ggggactagg gggtccccaa 1140

agtggcccac tttctgtggc tctgatcagc gcaggggcca gccagggaca tagccaggga 1200

ggggccacat cacttcccac tggaaatctc tgtggtctgc aagtgcttcc cagcccagaa 1260

caggggtgga ttccccaacc tcaacctcct ttcttctctg ctcccaaacc atgtcaggac 1320

caccttcctc tagagctcgg gagcccggag ggtcttcacc cactcctact ccagtatcag 1380

ctggcacggg ctccttcctg agagcaaagg tcaaggaccc cctctgtgaa ggctcagcag 1440

aggtgggatc ccacgccccc tcccggcccc tccctgccct ccattcaggg agaaacctct 1500

ccttcccgtg tgagaagggc cagagggtcc aggcatccca agtccagcgt gaagggccac 1560

agcccctctt ggctgccaag cacgcagatc ccatggacat ttggggaaag ggctccttgg 1620

gctgctggtg aacttctgtg gccaccacct cctgctcctg acctccctgg gagggtgcta 1680

tcagttctgt cctggccctt tcagttttat aagttggttt ccagccccca gtgtcctgac 1740

ttctgtctgc cacatgagga gggaggccct gcctgtgtgg gagggtggtt actgtgggtg 1800

gaatagtgga ggccttcaac tgattagaca aggcccgccc acatcttgga gggcatctgc 1860

cttactgatt aaaatgtcaa tgtaatctaa tgcaaaa 1897

<210> 4
<211> 2233
<212> DNA
<213> NM_006907.3 Homo sapiens pyrroline-5-carboxylate reductase 1
(PYCR1),transcript variant 1, mRNA.

<400> 4
gtacgacccg cgcacggtag ctgggttggg gtcggctccg ggaatggtgg ggcggcggtc 60

gccgggtggg gcgcgacggt agcccagttc tgggcggggt gggaacccag acgggggacc 120

cctaggcgcc gggccctccc tctccccgta cttttccccc ttcccagtca tttcttctgc 180

ggaacctcca cccctccagt gggtgcggga ccctaggcag caggccaggc accgccgctc 240

tgactggggg ccccgaagca gttaacgggc cggacagcaa agtggaaagt tagaccagct 300

gggaccaggg gaggtgggca cccggctgcg ggaggagcgg ccaggctggc actgcccccc 360

taacttgctc tcgatcctgc cggtctcgtc ccgcagagcc ggtgccatct gtgggggctt 420

tgggccaggg gtctccggac agcatgagcg tgggcttcat cggcgctggc cagctggctt 480

ttgccctggc caagggcttc acagcagcag gcgtcttggc tgcccacaag ataatggcta 540

gctccccaga catggacctg gccacagttt ctgctctcag gaagatgggg gtgaagttga 600

caccccacaa caaggagacg gtgcagcaca gtgatgtgct cttcctggct gtgaagccac 660

acatcatccc cttcatcctg gatgaaatag gcgccgacat tgaggacaga cacattgtgg 720

tgtcctgcgc ggccggcgtc accatcagct ccattgagaa gaagctgtca gcgtttcggc 780

cagcccccag ggtcatccgc tgcatgacca acactccagt cgtggtgcgg gagggggcca 840

ccgtgtatgc cacaggcacg cacgcccagg tggaggacgg gaggctcatg gagcagctgc 900

tgagcagcgt gggcttctgc acggaggtgg aagaggacct gattgatgcc gtcacggggc 960

tcagtggcag cggccccgcc tacgcattca cagccctgga tgccctggct gatgggggtg 1020

tgaagatggg acttccaagg cgcctggcag tccgcctcgg ggcccaggcc ctcctggggg 1080

ctgccaagat gctgctgcac tcagaacagc acccaggcca gctcaaggac aacgtcagct 1140

ctcctggtgg ggccaccatc catgccttgc atgtgctgga gagtgggggc ttccgctccc 1200

tgctcatcaa cgctgtggag gcctcctgca tccgcacacg ggagctgcag tccatggctg 1260

accaggagca ggtgtcacca gccgccatca agaagaccat cctggacaag gtgaagctgg 1320

actcccctgc agggaccgct ctgtcgcctt ctggccacac caagctgctc ccccgcagcc 1380

tggccccagc gggcaaggat tgacacgtcc tgcctgacca ccatcctgcc accaccttct 1440

cttctcttgt cactaggggg actagggggt ccccaaagtg gcccactttc tgtggctctg 1500

atcagcgcag gggccagcca gggacatagc cagggagggg ccacatcact tcccactgga 1560

aatctctgtg gtctgcaagt gcttcccagc ccagaacagg ggtggattcc ccaacctcaa 1620

cctcctttct tctctgctcc caaaccatgt caggaccacc ttcctctaga gctcgggagc 1680

ccggagggtc ttcacccact cctactccag tatcagctgg cacgggctcc ttcctgagag 1740

caaaggtcaa ggaccccctc tgtgaaggct cagcagaggt gggatcccac gccccctccc 1800

ggcccctccc tgccctccat tcagggagaa acctctcctt cccgtgtgag aagggccaga 1860

gggtccaggc atcccaagtc cagcgtgaag ggccacagcc cctcttggct gccaagcacg 1920

cagatcccat ggacatttgg ggaaagggct ccttgggctg ctggtgaact tctgtggcca 1980

ccacctcctg ctcctgacct ccctgggagg gtgctatcag ttctgtcctg gccctttcag 2040

ttttataagt tggtttccag cccccagtgt cctgacttct gtctgccaca tgaggaggga 2100

ggccctgcct gtgtgggagg gtggttactg tgggtggaat agtggaggcc ttcaactgat 2160

tagacaaggc ccgcccacat cttggagggc atctgcctta ctgattaaaa tgtcaatgta 2220

atctaatgca aaa 2233

<210> 5
<211> 1890
<212> DNA
<213> NM_153824.2 Homo sapiens pyrroline-5-carboxylate reductase 1 (PYCR1), transcript variant 2, mRNA

<400> 5
gtacgacccg cgcacggtag ctgggttggg gtcgggctcc gggaatggtg ggcggcggtc 60

gccgggtggg gcgcgacggt agcccagttc tgggcggggt gggaacccag acgggggacc 120

cctaggcgcc gggccctccc tctccccgta cttttccccc ttcccagtca tttcttctgc 180

ggaacctcca cccctccagt gggtgcggga ccctaggcag caggccaggc accgccgctc 240

tgactggggg ccccgaagca gttaacgggc cggacagcaa agtggaaagt tagaccagct 300

gggaccaggg gaggtgggca cccggctgcg ggaggagcgg ccaggctggc actgcccccc 360

taacttgctc tcgatcctgc cggtctcgtc ccgcagagcc ggtgccatct gtgggggctt 420

tgggccaggg gtctccggac agcatgagcg tgggcttcat cggcgctggc cagctggctt 480

ttgccctggc caagggcttc acagcagcag gcgtcttggc tgcccacaag ataatggcta 540

gctccccaga catggacctg gccacagttt ctgctctcag gaagatgggg gtgaagttga 600

caccccacaa caaggagacg gtgcagcaca gtgatgtgct cttcctggct gtgaagccac 660

acatcatccc cttcatcctg gatgaaatag gcgccgacat tgaggacaga cacattgtgg 720

tgtcctgcgc ggccggcgtc accatcagct ccattgagaa gaagctgtca gcgtttcggc 780

cagcccccag ggtcatccgc tgcatgacca acactccagt cgtggtgcgg gagggggcca 840

ccgtgtatgc cacaggcacg cacgcccagg tggaggacgg gaggctcatg gagcagctgc 900

tgagcagcgt gggcttctgc acggaggtgg aagaggacct gattgatgcc gtcacggggc 960

tcagtggcag cggccccgcc tacgcattca cagccctgga tgccctggct gatgggggtg 1020

tgaagatggg acttccaagg cgcctggcag tccgcctcgg ggcccaggcc ctcctggggg 1080

ctgccaagat gctgctgcac tcagaacagc acccaggcca gctcaaggac aacgtcagct 1140

ctcctggtgg ggccaccatc catgccttgc atgtgctgga gagtgggggc ttccgctccc 1200

tgctcatcaa cgctgtggag gcctcctgca tccgcacacg ggagctgcag tccatggctg 1260

accaggagca ggtgtcacca gccgccatca agaagaccat cctggacaag gaccaccttc 1320

ctctagagct cgggagcccg gagggtcttc acccactcct actccagtat cagctggcac 1380

gggctccttc ctgagagcaa aggtcaagga ccccctctgt gaaggctcag cagaggtggg 1440

atcccacgcc ccctcccggc ccctccctgc cctccattca gggagaaacc tctccttccc 1500

gtgtgagaag ggccagaggg tccaggcatc ccaagtccag cgtgaagggc cacagcccct 1560

cttggctgcc aagcacgcag atcccatgga catttgggga aagggctcct tgggctgctg 1620

gtgaacttct gtggccacca cctcctgctc ctgacctccc tgggagggtg ctatcagttc 1680

tgtcctggcc ctttcagttt tataagttgg tttccagccc ccagtgtcct gacttctgtc     1740

tgccacatga ggagggaggc cctgcctgtg tgggagggtg gttactgtgg gtggaatagt     1800

ggaggccttc aactgattag acaaggcccg cccacatctt ggagggcatc tgccttactg     1860

attaaaatgt caatgtaatc taatgcaaaa                                      1890


<210> 6
<211> 319
<212> PRT
<213> NP_001269209 PYRROLINE-5-CARBOXYLATE REDUCTASE 1, MITOCHONDRIAL
ISOFORM 1 [HOMOSAPIENS].


<400> 6
Met Ser Val Gly Phe Ile Gly Ala Gly Gln Leu Ala Phe Ala Leu Ala
1               5                   10                  15
Lys Gly Phe Thr Ala Ala Gly Val Leu Ala Ala His Lys Ile Met Ala
            20                  25                  30
Ser Ser Pro Asp Met Asp Leu Ala Thr Val Ser Ala Leu Arg Lys Met
        35                  40                  45
Gly Val Lys Leu Thr Pro His Asn Lys Glu Thr Val Gln His Ser Asp
    50                  55                  60
Val Leu Phe Leu Ala Val Lys Pro His Ile Ile Pro Phe Ile Leu Asp
65                  70                  75                  80
Glu Ile Gly Ala Asp Ile Glu Asp Arg His Ile Val Val Ser Cys Ala
                85                  90                  95
Ala Gly Val Thr Ile Ser Ser Ile Glu Lys Lys Leu Ser Ala Phe Arg
            100                 105                 110
Pro Ala Pro Arg Val Ile Arg Cys Met Thr Asn Thr Pro Val Val Val
        115                 120                 125
Arg Glu Gly Ala Thr Val Tyr Ala Thr Gly Thr His Ala Gln Val Glu
    130                 135                 140
Asp Gly Arg Leu Met Glu Gln Leu Leu Ser Ser Val Gly Phe Cys Thr
145                 150                 155                 160
Glu Val Glu Glu Asp Leu Ile Asp Ala Val Thr Gly Leu Ser Gly Ser
                165                 170                 175
Gly Pro Ala Tyr Ala Phe Thr Ala Leu Asp Ala Leu Ala Asp Gly Gly
            180                 185                 190
Val Lys Met Gly Leu Pro Arg Arg Leu Ala Val Arg Leu Gly Ala Gln
        195                 200                 205
Ala Leu Leu Gly Ala Ala Lys Met Leu Leu His Ser Glu Gln His Pro
    210                 215                 220
Gly Gln Leu Lys Asp Asn Val Ser Ser Pro Gly Gly Ala Thr Ile His
225                 230                 235                 240
Ala Leu His Val Leu Glu Ser Gly Gly Phe Arg Ser Leu Leu Ile Asn
                245                 250                 255
Ala Val Glu Ala Ser Cys Ile Arg Thr Arg Glu Leu Gln Ser Met Ala
            260                 265                 270
Asp Gln Glu Gln Val Ser Pro Ala Ala Ile Lys Lys Thr Ile Leu Asp
        275                 280                 285
Lys Val Lys Leu Asp Ser Pro Ala Gly Thr Ala Leu Ser Pro Ser Gly
    290                 295                 300
His Thr Lys Leu Leu Pro Arg Ser Leu Ala Pro Ala Gly Lys Asp
305                 310                 315

<210> 7
<211> 346
<212> PRT
<213> NP_001269210.1 pyrroline-5-carboxylate reductase 1, mitochondrial

isoform 5 [Homo

<400> 7

<210> 8
<211> 316
<212> PRT
<213> NP_722546.1 pyrroline-5-carboxylate reductase 1, mitochondrial isoform
2 [Homo sapiens].

<400> 8
Met Ser Val Gly Phe Ile Gly Ala Gly Gln Leu Ala Phe Ala Leu Ala
1               5                   10                  15
Lys Gly Phe Thr Ala Ala Gly Val Leu Ala Ala His Lys Ile Met Ala
            20                  25                  30
Ser Ser Pro Asp Met Asp Leu Ala Thr Val Ser Ala Leu Arg Lys Met
        35                  40                  45

Gly Val Lys Leu Thr Pro His Asn Lys Glu Thr Val Gln His Ser Asp
    50                  55                  60
Val Leu Phe Leu Ala Val Lys Pro His Ile Ile Pro Phe Ile Leu Asp
65                  70                  75                  80
Glu Ile Gly Ala Asp Ile Glu Asp Arg His Ile Val Val Ser Cys Ala
                85                  90                  95
Ala Gly Val Thr Ile Ser Ser Ile Glu Lys Lys Leu Ser Ala Phe Arg
            100                 105                 110
Pro Ala Pro Arg Val Ile Arg Cys Met Thr Asn Thr Pro Val Val Val
        115                 120                 125
Arg Glu Gly Ala Thr Val Tyr Ala Thr Gly Thr His Ala Gln Val Glu
    130                 135                 140
Asp Gly Arg Leu Met Glu Gln Leu Leu Ser Ser Val Gly Phe Cys Thr
145                 150                 155                 160
Glu Val Glu Glu Asp Leu Ile Asp Ala Val Thr Gly Leu Ser Gly Ser
                165                 170                 175
Gly Pro Ala Tyr Ala Phe Thr Ala Leu Asp Ala Leu Ala Asp Gly Gly
            180                 185                 190
Val Lys Met Gly Leu Pro Arg Arg Leu Ala Val Arg Leu Gly Ala Gln
        195                 200                 205
Ala Leu Leu Gly Ala Ala Lys Met Leu Leu His Ser Glu Gln His Pro
    210                 215                 220
Gly Gln Leu Lys Asp Asn Val Ser Ser Pro Gly Gly Ala Thr Ile His
225                 230                 235                 240
Ala Leu His Val Leu Glu Ser Gly Gly Phe Arg Ser Leu Leu Ile Asn
                245                 250                 255
Ala Val Glu Ala Ser Cys Ile Arg Thr Arg Glu Leu Gln Ser Met Ala
            260                 265                 270
Asp Gln Glu Gln Val Ser Pro Ala Ala Ile Lys Lys Thr Ile Leu Asp
        275                 280                 285
Lys Asp His Leu Pro Leu Glu Leu Gly Ser Pro Glu Gly Leu His Pro
    290                 295                 300
Leu Leu Leu Gln Tyr Gln Leu Ala Arg Ala Pro Ser
305                 310                 315

<210> 9
<211> 588
<212> PRT
<213> consensus sequence of SEQ ID Nos 1 - 5


<400> 9
   Gly Ala Gly Cys Cys Gly Gly Thr Gly Cys Cys Ala Thr Cys Thr Gly
   1               5                   10                  15
   Thr Gly Gly Gly Gly Gly Cys Thr Thr Thr Gly Gly Gly Cys Cys Ala
               20                  25                  30
   Gly Gly Gly Gly Thr Cys Thr Cys Cys Gly Gly Ala Cys Ala Gly Cys
           35                  40                  45
   Ala Thr Gly Ala Gly Cys Gly Thr Gly Gly Gly Cys Thr Thr Cys Ala
       50                  55                  60
   Thr Cys Gly Gly Cys Gly Cys Thr Gly Gly Cys Cys Ala Gly Cys Thr
   65                  70                  75                  80
   Gly Gly Cys Thr Thr Thr Thr Gly Cys Cys Cys Thr Gly Gly Cys Cys
                   85                  90                  95
   Ala Ala Gly Gly Gly Cys Thr Thr Cys Ala Cys Ala Gly Cys Ala Gly
               100                 105                 110
   Cys Ala Gly Gly Cys Gly Thr Cys Thr Thr Gly Gly Cys Thr Gly Cys
           115                 120                 125
   Cys Cys Ala Cys Ala Ala Gly Ala Thr Ala Ala Thr Gly Gly Cys Thr
       130                 135                 140
   Ala Gly Cys Thr Cys Cys Cys Cys Ala Gly Ala Cys Ala Thr Gly Gly
   145                 150                 155                 160
   Ala Cys Cys Thr Gly Gly Cys Cys Ala Cys Ala Gly Thr Thr Thr Cys
   165 170 175
   Thr Gly Cys Thr Cys Thr Cys Ala Gly Gly Ala Ala Gly Ala Thr Gly
   180 185 190
   Gly Gly Gly Gly Thr Gly Ala Ala Gly Thr Thr Gly Ala Cys Ala Cys
   195 200 205
   Cys Cys Cys Ala Cys Ala Ala Cys Ala Ala Gly Gly Ala Gly Ala Cys
   210 215 220
   Gly Gly Thr Gly Cys Ala Gly Cys Ala Cys Ala Gly Thr Gly Ala Thr
   225 230 235 240
   Gly Thr Gly Cys Thr Cys Thr Thr Cys Cys Thr Gly Gly Cys Thr Gly
   245 250 255
   Thr Gly Ala Ala Gly Cys Cys Ala Cys Ala Cys Ala Thr Cys Ala Thr
   260 265 270
   Cys Cys Cys Cys Thr Thr Cys Ala Thr Cys Cys Thr Gly Gly Ala Thr
   275 280 285
   Gly Ala Ala Ala Thr Ala Gly Gly Cys Gly Cys Cys Gly Ala Cys Ala
   290 295 300
   Thr Thr Gly Ala Gly Gly Ala Cys Ala Gly Ala Cys Ala Cys Ala Thr
   305 310 315 320
   Thr Gly Thr Gly Gly Thr Gly Thr Cys Cys Thr Gly Cys Gly Cys Gly
   325 330 335
   Gly Cys Cys Gly Gly Cys Gly Thr Cys Ala Cys Cys Ala Thr Cys Ala
   340 345 350
   Gly Cys Thr Cys Cys Ala Thr Thr Gly Ala Gly Ala Ala Gly Ala Ala
   355 360 365
   Gly Cys Thr Gly Thr Cys Ala Gly Cys Gly Thr Thr Thr Cys Gly Gly
   370 375 380
   Cys Cys Ala Gly Cys Cys Cys Cys Cys Ala Gly Gly Gly Thr Cys Ala
   385 390 395 400
   Thr Cys Cys Gly Cys Thr Gly Cys Ala Thr Gly Ala Cys Cys Ala Ala
   405 410 415
   Cys Ala Cys Thr Cys Cys Ala Gly Thr Cys Gly Thr Gly Gly Thr Gly
   420 425 430
   Cys Gly Gly Gly Ala Gly Gly Gly Gly Gly Cys Cys Ala Cys Cys Gly
   435 440 445
   Thr Gly Thr Ala Thr Gly Cys Cys Ala Cys Ala Gly Gly Cys Ala Cys
   450 455 460
   Gly Cys Ala Cys Gly Cys Cys Cys Ala Gly Gly Thr Gly Gly Ala Gly
   465 470 475 480
   Gly Ala Cys Gly Gly Gly Ala Gly Gly Cys Thr Cys Ala Thr Gly Gly
   485 490 495
   Ala Gly Cys Ala Gly Cys Thr Gly Cys Thr Gly Ala Gly Cys Ala Gly
   500 505 510
   Cys Gly Thr Gly Gly Gly Cys Thr Thr Cys Thr Gly Cys Ala Cys Gly
   515 520 525
   Gly Ala Gly Gly Thr Gly Gly Ala Ala Gly Ala Gly Gly Ala Cys Cys
   530 535 540
   Thr Gly Ala Thr Thr Gly Ala Thr Gly Cys Cys Gly Thr Cys Ala Cys
   545 550 555 560
   Gly Gly Gly Gly Cys Thr Cys Ala Gly Thr Gly Gly Cys Ala Gly Cys
   565 570 575
   Gly Gly Cys Cys Cys Cys Gly Cys Cys Thr Ala Cys
   580 585

<210> 10
<211> 290
<212> PRT
<213> Consensus sequence of SEQ ID Nos 6 - 8

<400> 10
Met Ser Val Gly Phe Ile Gly Ala Gly Gln Leu Ala Phe Ala Leu Ala
1               5                   10                  15

Lys Gly Phe Thr Ala Ala Gly Val Leu Ala Ala His Lys Ile Met Ala
            20                  25                  30
Ser Ser Pro Asp Met Asp Leu Ala Thr Val Ser Ala Leu Arg Lys Met
        35                  40                  45
Gly Val Lys Leu Thr Pro His Asn Lys Glu Thr Val Gln His Ser Asp
    50                  55                  60
Val Leu Phe Leu Ala Val Lys Pro His Ile Ile Pro Phe Ile Leu Asp
65                  70                  75                  80
Glu Ile Gly Ala Asp Ile Glu Asp Arg His Ile Val Val Ser Cys Ala
                85                  90                  95
Ala Gly Val Thr Ile Ser Ser Ile Glu Lys Lys Leu Ser Ala Phe Arg
            100                 105                 110
Pro Ala Pro Arg Val Ile Arg Cys Met Thr Asn Thr Pro Val Val Val
        115                 120                 125
Arg Glu Gly Ala Thr Val Tyr Ala Thr Gly Thr His Ala Gln Val Glu
    130                 135                 140
Asp Gly Arg Leu Met Glu Gln Leu Leu Ser Ser Val Gly Phe Cys Thr
145                 150                 155                 160
Glu Val Glu Glu Asp Leu Ile Asp Ala Val Thr Gly Leu Ser Gly Ser
                165                 170                 175
Gly Pro Ala Tyr Ala Phe Thr Ala Leu Asp Ala Leu Ala Asp Gly Gly
            180                 185                 190
Val Lys Met Gly Leu Pro Arg Arg Leu Ala Val Arg Leu Gly Ala Gln
        195                 200                 205
Ala Leu Leu Gly Ala Ala Lys Met Leu Leu His Ser Glu Gln His Pro
    210                 215                 220
Gly Gln Leu Lys Asp Asn Val Ser Ser Pro Gly Gly Ala Thr Ile His
225                 230                 235                 240
Ala Leu His Val Leu Glu Ser Gly Gly Phe Arg Ser Leu Leu Ile Asn
                245                 250                 255
Ala Val Glu Ala Ser Cys Ile Arg Thr Arg Glu Leu Gln Ser Met Ala
            260                 265                 270
Asp Gln Glu Gln Val Ser Pro Ala Ala Ile Lys Lys Thr Ile Leu Asp
        275                 280                 285
Lys Val
    290

<210> 11
<211> 21
<212> DNA
<213> Rat PYCR 1.1

<400> 11
gctgccataa agaagactgt a                                                21

<210> 12
<211> 21
<212> DNA
<213> Rat PYCR 1.2

<400> 12
gaacctgaca ccccacaaca a                                                21

<210> 13
<211> 21
<212> DNA
<213> Rat PYCR 1.3

<400> 13
ggcagccaag atgctactag a                                                21